# EUROPEAN PATENT APPLICATION

(11) **EP 3 239 295 A1**
(43) Date of publication of application: **01.11.2017**
(21) Application number: 15867083.6
(22) Date of filing: 14.12.2015
(51) Int. Cl.: C12N 15/09, A61K 39/395, A61P 25/28, C07K 16/18, C12N 15/02, G01N 33/53

(54) **ANTIBODY HIGHLY SPECIFICALLY RECOGNIZING TURN STRUCTURE AT 22- AND 23-POSITIONS IN AMYLOID BETA**

(30) Priority: 12.12.2014 JP 2014251898; 22.05.2015 JP 2015104411
(71) Applicant: Kyoto University, Kyoto-shi, Kyoto 606-8501 (JP); National University Corporation Chiba University, Chiba-shi, Chiba 263-0022 (JP); Immuno-Biological Laboratories Co., Ltd., Fujioka-shi Gunma 375-0005 (JP)
(72) Inventor: IRIE, Kazuhiro, Kyoto-shi Kyoto 606-8501 (JP); MURAKAMI, Kazuma, Kyoto-shi Kyoto 606-8501 (JP); SHIMIZU, Takahiko, Chiba-shi Chiba 260-8670 (JP); IZUO, Naotaka, Chiba-shi Chiba 260-8670 (JP); SEITO, Tsutomu, Fujioka-shi Gunma 375-0005 (JP)
(74) Representative: J A Kemp
(86) International application number: PCT/JP2015/006224
(87) International publication number: WO 2016/092865

(57) **Abstract**

Provided is an antibody or an immunologically reactive fragment thereof which targets exclusively an Aβ having a specific turn structure of Aβ (a toxic conformer of Aβ). Also provided are a medicinal composition comprising, as an active ingredient, an antibody that specifically recognizes a toxic conformer of Aβ, a kit for assaying a toxic conformer of Aβ, a diagnostic agent for Alzheimer's disease, etc.

SEQUENCE LISTING

<110>  Immuno-Biological Laboratories Co., Ltd.
       Kyoto University
       National University Corporation Chiba University

<120>  An antibody that very specifically recognize turn structure at
       positions 22 and 23 of amyloid beta

<130>  PW15017IB

<150>  JP2014-251898
<151>  2012-12-12

<150>  JP2015-104411
<151>  2015-05-22

<160>  14

<170>  PatentIn version 3.5

<210>  1
<211>  1483
<212>  DNA
<213>  Artificial Sequence

<220>
<223>  antibody


<220>
<221>  CDS
<222>  (69)..(1454)

<220>
<221>  sig_peptide
<222>  (69)..(126)

<400>  1
ctacagcgtt tacttaagct tggtaccgag ctcggatcca ctagtccagt gtggtggaat      60

tcctaacc atg gga tgg agc tgg atc ttt ctc ctc ttc ctg tca gga act      110
         Met Gly Trp Ser Trp Ile Phe Leu Leu Phe Leu Ser Gly Thr
         1               5                   10

gca ggt gtc ctc tct gag gtc cag ctg caa cag tct gga cct gag ctg      158
Ala Gly Val Leu Ser Glu Val Gln Leu Gln Gln Ser Gly Pro Glu Leu
15                  20                  25                  30

gtg aag cct ggg gct tca gtg aag ata ttc tgc aag gct tct gga tac      206
Val Lys Pro Gly Ala Ser Val Lys Ile Phe Cys Lys Ala Ser Gly Tyr
                35                  40                  45

act ttc act gac tac aat atg gac tgg gtg aag cag agt cat gga aag      254
Thr Phe Thr Asp Tyr Asn Met Asp Trp Val Lys Gln Ser His Gly Lys
            50                  55                  60

aga ctt gag tgg att gga gat att aat cct aac act ggt gtt act atc      302
Arg Leu Glu Trp Ile Gly Asp Ile Asn Pro Asn Thr Gly Val Thr Ile
        65                  70                  75

gac aat ccg aag ttc aag ggc aag gcc aca ttg act gta gac gag tcc      350
Asp Asn Pro Lys Phe Lys Gly Lys Ala Thr Leu Thr Val Asp Glu Ser

80                  85                  90

tcc agt aca gtc tac atg gag ctc cgc agc ctg tca tct gaa gac act          398
Ser Ser Thr Val Tyr Met Glu Leu Arg Ser Leu Ser Ser Glu Asp Thr
95                  100                 105                 110

gcc gtc tat tac tgt gca aga tta ccc gat aac tat gtt atg gac tat          446
Ala Val Tyr Tyr Cys Ala Arg Leu Pro Asp Asn Tyr Val Met Asp Tyr
                115                 120                 125

tgg ggt caa gga acc tca gtc acc gtc tcc gca gcc aaa acg aca ccc          494
Trp Gly Gln Gly Thr Ser Val Thr Val Ser Ala Ala Lys Thr Thr Pro
            130                 135                 140

cca tct gtc tat cca ctg gcc cct gga tct gct gcc caa act aac tcc          542
Pro Ser Val Tyr Pro Leu Ala Pro Gly Ser Ala Ala Gln Thr Asn Ser
        145                 150                 155

atg gtg acc ctg gga tgc ctg gtc aag ggc tat ttc cct gag cca gtg          590
Met Val Thr Leu Gly Cys Leu Val Lys Gly Tyr Phe Pro Glu Pro Val
    160                 165                 170

aca gtg acc tgg aac tct gga tcc ctg tcc agc ggt gtg cac acc ttc          638
Thr Val Thr Trp Asn Ser Gly Ser Leu Ser Ser Gly Val His Thr Phe
175                 180                 185                 190

cca gct gtc ctg cag tct gac ctc tac act ctg agc agc tca gtg act          686
Pro Ala Val Leu Gln Ser Asp Leu Tyr Thr Leu Ser Ser Ser Val Thr
                195                 200                 205

gtc ccc tcc agc acc tgg ccc agc cag acc gtc acc tgc aac gtt gcc          734
Val Pro Ser Ser Thr Trp Pro Ser Gln Thr Val Thr Cys Asn Val Ala
            210                 215                 220

cac ccg gcc agc agc acc aaa gtg gac aag aaa att gtg ccc agg gat          782
His Pro Ala Ser Ser Thr Lys Val Asp Lys Lys Ile Val Pro Arg Asp
        225                 230                 235

tgt ggt tgt aag cct tgc ata tgt aca gtc cca gaa gta tca tct gtc          830
Cys Gly Cys Lys Pro Cys Ile Cys Thr Val Pro Glu Val Ser Ser Val
    240                 245                 250

ttc atc ttc ccc cca aag ccc aag gat gtg ctc acc att act ctg act          878
Phe Ile Phe Pro Pro Lys Pro Lys Asp Val Leu Thr Ile Thr Leu Thr
255                 260                 265                 270

cct aag gtc acg tgt gtt gtg gta gac atc agc aag gat gat ccc gag          926
Pro Lys Val Thr Cys Val Val Val Asp Ile Ser Lys Asp Asp Pro Glu
                275                 280                 285

gtc cag ttc agc tgg ttt gta gat gat gtg gag gtg cac aca gct cag          974
Val Gln Phe Ser Trp Phe Val Asp Asp Val Glu Val His Thr Ala Gln
            290                 295                 300

acg aaa ccc cgg gag gag cag atc aac agc act ttc cgt tca gtc agt          1022
Thr Lys Pro Arg Glu Glu Gln Ile Asn Ser Thr Phe Arg Ser Val Ser
        305                 310                 315

gaa ctt ccc atc atg cac cag gac tgg ctc aat ggc aag gag ttc aaa          1070
Glu Leu Pro Ile Met His Gln Asp Trp Leu Asn Gly Lys Glu Phe Lys
    320                 325                 330

tgc agg gtc aac agt gca gct ttc cct gcc ccc atc gag aaa acc atc          1118

Cys Arg Val Asn Ser Ala Ala Phe Pro Ala Pro Ile Glu Lys Thr Ile
335                 340                 345                 350

tcc aaa acc aaa ggc aga ccg aag gct cca cag gtg tac acc att cca      1166
Ser Lys Thr Lys Gly Arg Pro Lys Ala Pro Gln Val Tyr Thr Ile Pro
                355                 360                 365

cct ccc aag gag cag atg gcc aag gat aaa gtc agt ctg acc tgc atg      1214
Pro Pro Lys Glu Gln Met Ala Lys Asp Lys Val Ser Leu Thr Cys Met
            370                 375                 380

ata aca aac ttc ttc cct gaa gac att act gtg gag tgg cag tgg aat      1262
Ile Thr Asn Phe Phe Pro Glu Asp Ile Thr Val Glu Trp Gln Trp Asn
        385                 390                 395

ggg cag cca gcg gag aac tac aag aac act cag ccc atc atg gac aca      1310
Gly Gln Pro Ala Glu Asn Tyr Lys Asn Thr Gln Pro Ile Met Asp Thr
    400                 405                 410

gat ggc tct tac ttc gtc tac agc aag ctc aat gtg cag aag agc aac      1358
Asp Gly Ser Tyr Phe Val Tyr Ser Lys Leu Asn Val Gln Lys Ser Asn
415                 420                 425                 430

tgg gag gca gga aat act ttc acc tgc tct gtg tta cat gag ggc ctg      1406
Trp Glu Ala Gly Asn Thr Phe Thr Cys Ser Val Leu His Glu Gly Leu
                435                 440                 445

cac aac cac cat act gag aag agc ctc tcc cac tct cct ggt aaa taa      1454
His Asn His His Thr Glu Lys Ser Leu Ser His Ser Pro Gly Lys
            450                 455                 460

gcggccgctc gagtctagag tcccgtatg                                      1483


<210>  2
<211>  461
<212>  PRT
<213>  Artificial Sequence

<220>
<223>  Synthetic Construct

<400>  2

Met Gly Trp Ser Trp Ile Phe Leu Leu Phe Leu Ser Gly Thr Ala Gly
1               5                   10                  15


Val Leu Ser Glu Val Gln Leu Gln Gln Ser Gly Pro Glu Leu Val Lys
            20                  25                  30


Pro Gly Ala Ser Val Lys Ile Phe Cys Lys Ala Ser Gly Tyr Thr Phe
        35                  40                  45


Thr Asp Tyr Asn Met Asp Trp Val Lys Gln Ser His Gly Lys Arg Leu
    50                  55                  60


Glu Trp Ile Gly Asp Ile Asn Pro Asn Thr Gly Val Thr Ile Asp Asn
65                  70                  75                  80

Pro Lys Phe Lys Gly Lys Ala Thr Leu Thr Val Asp Glu Ser Ser Ser
                85                  90                  95

Thr Val Tyr Met Glu Leu Arg Ser Leu Ser Ser Glu Asp Thr Ala Val
            100                 105                 110

Tyr Tyr Cys Ala Arg Leu Pro Asp Asn Tyr Val Met Asp Tyr Trp Gly
        115                 120                 125

Gln Gly Thr Ser Val Thr Val Ser Ala Ala Lys Thr Thr Pro Pro Ser
    130                 135                 140

Val Tyr Pro Leu Ala Pro Gly Ser Ala Ala Gln Thr Asn Ser Met Val
145                 150                 155                 160

Thr Leu Gly Cys Leu Val Lys Gly Tyr Phe Pro Glu Pro Val Thr Val
                165                 170                 175

Thr Trp Asn Ser Gly Ser Leu Ser Ser Gly Val His Thr Phe Pro Ala
            180                 185                 190

Val Leu Gln Ser Asp Leu Tyr Thr Leu Ser Ser Ser Val Thr Val Pro
        195                 200                 205

Ser Ser Thr Trp Pro Ser Gln Thr Val Thr Cys Asn Val Ala His Pro
    210                 215                 220

Ala Ser Ser Thr Lys Val Asp Lys Lys Ile Val Pro Arg Asp Cys Gly
225                 230                 235                 240

Cys Lys Pro Cys Ile Cys Thr Val Pro Glu Val Ser Ser Val Phe Ile
                245                 250                 255

Phe Pro Pro Lys Pro Lys Asp Val Leu Thr Ile Thr Leu Thr Pro Lys
            260                 265                 270

Val Thr Cys Val Val Val Asp Ile Ser Lys Asp Asp Pro Glu Val Gln
        275                 280                 285

Phe Ser Trp Phe Val Asp Asp Val Glu Val His Thr Ala Gln Thr Lys
    290                 295                 300

Pro Arg Glu Glu Gln Ile Asn Ser Thr Phe Arg Ser Val Ser Glu Leu
305                 310                 315                 320

Pro Ile Met His Gln Asp Trp Leu Asn Gly Lys Glu Phe Lys Cys Arg
                325                 330                 335

Val Asn Ser Ala Ala Phe Pro Ala Pro Ile Glu Lys Thr Ile Ser Lys
            340                 345                 350

Thr Lys Gly Arg Pro Lys Ala Pro Gln Val Tyr Thr Ile Pro Pro Pro
        355                 360                 365

Lys Glu Gln Met Ala Lys Asp Lys Val Ser Leu Thr Cys Met Ile Thr
    370                 375                 380

Asn Phe Phe Pro Glu Asp Ile Thr Val Glu Trp Gln Trp Asn Gly Gln
385                 390                 395                 400

Pro Ala Glu Asn Tyr Lys Asn Thr Gln Pro Ile Met Asp Thr Asp Gly
                405                 410                 415

Ser Tyr Phe Val Tyr Ser Lys Leu Asn Val Gln Lys Ser Asn Trp Glu
            420                 425                 430

Ala Gly Asn Thr Phe Thr Cys Ser Val Leu His Glu Gly Leu His Asn
        435                 440                 445

His His Thr Glu Lys Ser Leu Ser His Ser Pro Gly Lys
    450                 455                 460

<210> 3
<211> 442
<212> PRT
<213> Artificial Sequence

<220>
<223> Hc without signal peptide

<400> 3

Glu Val Gln Leu Gln Gln Ser Gly Pro Glu Leu Val Lys Pro Gly Ala
1               5                   10                  15

Ser Val Lys Ile Phe Cys Lys Ala Ser Gly Tyr Thr Phe Thr Asp Tyr
            20                  25                  30

Asn Met Asp Trp Val Lys Gln Ser His Gly Lys Arg Leu Glu Trp Ile
        35                  40                  45

Gly Asp Ile Asn Pro Asn Thr Gly Val Thr Ile Asp Asn Pro Lys Phe
    50                  55                  60

Lys Gly Lys Ala Thr Leu Thr Val Asp Glu Ser Ser Ser Thr Val Tyr
65                  70                  75                  80

Met Glu Leu Arg Ser Leu Ser Ser Glu Asp Thr Ala Val Tyr Tyr Cys
                85                  90                  95

Ala Arg Leu Pro Asp Asn Tyr Val Met Asp Tyr Trp Gly Gln Gly Thr
            100                 105                 110

Ser Val Thr Val Ser Ala Ala Lys Thr Thr Pro Pro Ser Val Tyr Pro
        115                 120                 125

Leu Ala Pro Gly Ser Ala Ala Gln Thr Asn Ser Met Val Thr Leu Gly
    130                 135                 140

Cys Leu Val Lys Gly Tyr Phe Pro Glu Pro Val Thr Val Thr Trp Asn
145                 150                 155                 160

Ser Gly Ser Leu Ser Ser Gly Val His Thr Phe Pro Ala Val Leu Gln
                165                 170                 175

Ser Asp Leu Tyr Thr Leu Ser Ser Ser Val Thr Val Pro Ser Gln His
            180                 185                 190

Leu Ala Gln Pro Asp Arg His Cys Asn Val Ala His Pro Ala Ser Ser
        195                 200                 205

Thr Lys Val Asp Lys Lys Ile Val Pro Arg Asp Cys Gly Cys Lys Pro
    210                 215                 220

Cys Ile Cys Thr Val Pro Glu Val Ser Ser Val Phe Ile Phe Pro Pro
225                 230                 235                 240

Lys Pro Lys Asp Val Leu Thr Ile Thr Leu Thr Pro Lys Val Thr Cys
                245                 250                 255

Val Val Val Asp Ile Ser Lys Asp Asp Pro Glu Val Gln Phe Ser Trp
            260                 265                 270

Phe Val Asp Asp Val Glu Val His Thr Ala Gln Thr Lys Pro Arg Glu
        275                 280                 285

Glu Gln Ile Asn Ser Thr Phe Arg Ser Val Ser Glu Leu Pro Ile Met
    290                 295                 300

His Gln Asp Trp Leu Asn Gly Lys Glu Phe Lys Cys Arg Val Asn Ser
305                 310                 315                 320

Ala Ala Phe Pro Ala Pro Ile Glu Lys Thr Ile Ser Lys Thr Lys Gly
                325                 330                 335

Arg Pro Lys Ala Pro Gln Val Tyr Thr Ile Pro Pro Pro Lys Glu Gln
            340                 345                 350

Met Ala Lys Asp Lys Val Ser Leu Thr Cys Met Ile Thr Asn Phe Phe
        355                 360                 365

Pro Glu Asp Ile Thr Val Glu Trp Gln Trp Asn Gly Gln Pro Ala Glu
    370                 375                 380

Asn Tyr Lys Asn Thr Gln Pro Ile Met Asp Thr Asp Gly Ser Tyr Phe
385                 390                 395                 400

Val Tyr Ser Lys Leu Asn Val Gln Lys Ser Asn Trp Glu Ala Gly Asn
                405                 410                 415

Thr Phe Thr Cys Ser Val Leu His Glu Gly Leu His Asn His His Thr
            420                 425                 430

Glu Lys Ser Leu Ser His Ser Pro Gly Lys
        435                 440

<210> 4
<211> 118
<212> PRT
<213> Artificial Sequence

<220>
<223> VH

<400> 4

Glu Val Gln Leu Gln Gln Ser Gly Pro Glu Leu Val Lys Pro Gly Ala
1               5                   10                  15

Ser Val Lys Ile Phe Cys Lys Ala Ser Gly Tyr Thr Phe Thr Asp Tyr
            20                  25                  30

Asn Met Asp Trp Val Lys Gln Ser His Gly Lys Arg Leu Glu Trp Ile
        35                  40                  45

Gly Asp Ile Asn Pro Asn Thr Gly Val Thr Ile Asp Asn Pro Lys Phe
    50                  55                  60

Lys Gly Lys Ala Thr Leu Thr Val Asp Glu Ser Ser Ser Thr Val Tyr
65                  70                  75                  80

Met Glu Leu Arg Ser Leu Ser Ser Glu Asp Thr Ala Val Tyr Tyr Cys
                85                  90                  95

Ala Arg Leu Pro Asp Asn Tyr Val Met Asp Tyr Trp Gly Gln Gly Thr
            100                 105                 110

Ser Val Thr Val Ser Ala
        115

<210>  5
<211>  5
<212>  PRT
<213>  Artificial Sequence

<220>
<223>  CDRH1

<400>  5

Asp Tyr Asn Met Asp
1               5

<210>  6
<211>  17
<212>  PRT
<213>  Artificial Sequence

<220>
<223>  CDRH2

<400>  6

Asp Ile Asn Pro Asn Thr Gly Val Thr Ile Asp Asn Pro Lys Phe Lys
1               5                   10                  15

Gly

<210>  7
<211>  9
<212>  PRT
<213>  Artificial Sequence

<220>
<223>  CDRH3

<400>  7

Leu Pro Asp Asn Tyr Val Met Asp Tyr
1               5

<210>  8
<211>  820
<212>  DNA
<213>  Artificial Sequence

<220>
<223>  antibody

<220>
<221>  CDS
<222>  (73)..(789)

<220>
<221>  sig_peptide
<222>  (73)..(92)

<400>  8
cataccaagc gttttactta gcttggtacc gagctcggat ccactagtcc agtgtgggtg      60

gaattcctca aa atg aag ttg cct gtt agg ctg ttg gtg ctg atg ttc tgg      111
              Met Lys Leu Pro Val Arg Leu Leu Val Leu Met Phe Trp
              1               5                   10

att cct gct tcc agc agt gat gtt gtg atg acc caa act cca ctc tcc      159
Ile Pro Ala Ser Ser Ser Asp Val Val Met Thr Gln Thr Pro Leu Ser
    15                  20                  25

ctg cct gtc agt ctt gga gat caa gcc tcc atc tct tgc aga tgt agt      207
Leu Pro Val Ser Leu Gly Asp Gln Ala Ser Ile Ser Cys Arg Cys Ser
30                  35                  40                  45

cag agc ctt gta cac aga aat gga aac acc aat tta cat tgg tac ctg      255
Gln Ser Leu Val His Arg Asn Gly Asn Thr Asn Leu His Trp Tyr Leu
                50                  55                  60

cag aag tca ggc cag tct cca aag ctc ctg atc tac aaa gtt tcc aac      303
Gln Lys Ser Gly Gln Ser Pro Lys Leu Leu Ile Tyr Lys Val Ser Asn
            65                  70                  75

cga ttt tct ggg gtc cca gac agg ttc agt ggc agt gga tca ggg aca      351
Arg Phe Ser Gly Val Pro Asp Arg Phe Ser Gly Ser Gly Ser Gly Thr
        80                  85                  90

gat ttc aca ctc aag atc agc aga gtg gag gct gag gat ctg gga gtt      399
Asp Phe Thr Leu Lys Ile Ser Arg Val Glu Ala Glu Asp Leu Gly Val
    95                  100                 105

tat ttc tgc tct caa agt aca tat gtt ccg ctc acg ttc ggt gtt ggg      447
Tyr Phe Cys Ser Gln Ser Thr Tyr Val Pro Leu Thr Phe Gly Val Gly
110                 115                 120                 125

acc aag ctg gag ctg aaa cgg gct gat gct gca cca act gta tcc atc      495
Thr Lys Leu Glu Leu Lys Arg Ala Asp Ala Ala Pro Thr Val Ser Ile
                130                 135                 140

ttc cca cca tcc agt gag cag tta aca tct gga ggt gcc tca gtc gtg      543
Phe Pro Pro Ser Ser Glu Gln Leu Thr Ser Gly Gly Ala Ser Val Val
            145                 150                 155

tgc ttc ttg aac aac ttc tac ccc aaa gac atc aat gtc aag tgg aag      591
Cys Phe Leu Asn Asn Phe Tyr Pro Lys Asp Ile Asn Val Lys Trp Lys
        160                 165                 170

att gat ggc agt gaa cga caa aat ggc gtc ctg aac agt tgg act gat      639
Ile Asp Gly Ser Glu Arg Gln Asn Gly Val Leu Asn Ser Trp Thr Asp
    175                 180                 185

cag gac agc aaa gac agc acc tac agc atg agc agc acc ctc acg ttg      687
Gln Asp Ser Lys Asp Ser Thr Tyr Ser Met Ser Ser Thr Leu Thr Leu
190                 195                 200                 205

acc aag gac gag tat gaa cga cat aac agc tat acc tgt gag gcc act 735
Thr Lys Asp Glu Tyr Glu Arg His Asn Ser Tyr Thr Cys Glu Ala Thr
                210                 215                 220

cac aag aca tca act tca ccc att gtc aag agc ttc aac agg aat gag 783
His Lys Thr Ser Thr Ser Pro Ile Val Lys Ser Phe Asn Arg Asn Glu
            225                 230                 235

tgt tag gcgggccgct cgagtctaga gggcccgttt a 820
Cys

<210> 9
<211> 238
<212> PRT
<213> Artificial Sequence

<220>
<223> Synthetic Construct

<400> 9

Met Lys Leu Pro Val Arg Leu Leu Val Leu Met Phe Trp Ile Pro Ala
1               5                   10                  15

Ser Ser Ser Asp Val Val Met Thr Gln Thr Pro Leu Ser Leu Pro Val
            20                  25                  30

Ser Leu Gly Asp Gln Ala Ser Ile Ser Cys Arg Cys Ser Gln Ser Leu
        35                  40                  45

Val His Arg Asn Gly Asn Thr Asn Leu His Trp Tyr Leu Gln Lys Ser
    50                  55                  60

Gly Gln Ser Pro Lys Leu Leu Ile Tyr Lys Val Ser Asn Arg Phe Ser
65                  70                  75                  80

Gly Val Pro Asp Arg Phe Ser Gly Ser Gly Ser Gly Thr Asp Phe Thr
                85                  90                  95

Leu Lys Ile Ser Arg Val Glu Ala Glu Asp Leu Gly Val Tyr Phe Cys
            100                 105                 110

Ser Gln Ser Thr Tyr Val Pro Leu Thr Phe Gly Val Gly Thr Lys Leu
        115                 120                 125

Glu Leu Lys Arg Ala Asp Ala Ala Pro Thr Val Ser Ile Phe Pro Pro
    130                 135                 140

Ser Ser Glu Gln Leu Thr Ser Gly Gly Ala Ser Val Val Cys Phe Leu
145                 150                 155                 160

Asn Asn Phe Tyr Pro Lys Asp Ile Asn Val Lys Trp Lys Ile Asp Gly
                165                 170                 175

Ser Glu Arg Gln Asn Gly Val Leu Asn Ser Trp Thr Asp Gln Asp Ser
            180                 185                 190

Lys Asp Ser Thr Tyr Ser Met Ser Ser Thr Leu Thr Leu Thr Lys Asp
        195                 200                 205

Glu Tyr Glu Arg His Asn Ser Tyr Thr Cys Glu Ala Thr His Lys Thr
    210                 215                 220

Ser Thr Ser Pro Ile Val Lys Ser Phe Asn Arg Asn Glu Cys
225                 230                 235

<210>  10
<211>  219
<212>  PRT
<213>  Artificial Sequence

<220>
<223>  Lc without sigal peptide

<400>  10

Asp Val Val Met Thr Gln Thr Pro Leu Ser Leu Pro Val Ser Leu Gly
1               5                   10                  15

Asp Gln Ala Ser Ile Ser Cys Arg Cys Ser Gln Ser Leu Val His Arg
            20                  25                  30

Asn Gly Asn Thr Asn Leu His Trp Tyr Leu Gln Lys Ser Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr Tyr Val Pro Leu Thr Phe Gly Val Gly Thr Lys Leu Glu Leu Lys
            100                 105                 110

Arg Ala Asp Ala Ala Pro Thr Val Ser Ile Phe Pro Pro Ser Ser Glu
        115                 120                 125

Gln Leu Thr Ser Gly Gly Ala Ser Val Val Cys Phe Leu Asn Asn Phe
    130                 135                 140

Tyr Pro Lys Asp Ile Asn Val Lys Trp Lys Ile Asp Gly Ser Glu Arg
145                 150                 155                 160

Gln Asn Gly Val Leu Asn Ser Trp Thr Asp Gln Asp Ser Lys Asp Ser
                165                 170                 175

Thr Tyr Ser Met Ser Ser Thr Leu Thr Leu Thr Lys Asp Glu Tyr Glu
            180                 185                 190

Arg His Asn Ser Tyr Thr Cys Glu Ala Thr His Lys Thr Ser Thr Ser
        195                 200                 205

Pro Ile Val Lys Ser Phe Asn Arg Asn Glu Cys
    210                 215

<210>  11
<211>  112
<212>  PRT
<213>  Artificial Sequence

<220>
<223>  VL

<400>  11

Asp Val Val Met Thr Gln Thr Pro Leu Ser Leu Pro Val Ser Leu Gly
1               5                   10                  15

Asp Gln Ala Ser Ile Ser Cys Arg Cys Ser Gln Ser Leu Val His Arg
            20                  25                  30

Asn Gly Asn Thr Asn Leu His Trp Tyr Leu Gln Lys Ser Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr Tyr Val Pro Leu Thr Phe Gly Val Gly Thr Lys Leu Glu Leu Lys
            100                 105                 110

<210>  12

<211>  16
<212>  PRT
<213>  Artificial Sequence

<220>
<223>  CDRL1

<400>  12

Arg Cys Ser Gln Ser Leu Val His Arg Asn Gly Asn Thr Asn Leu His
1               5                   10                  15


<210>  13
<211>  7
<212>  PRT
<213>  Artificial Sequence

<220>
<223>  CDRL2

<400>  13

Lys Val Ser Asn Arg Phe Ser
1               5


<210>  14
<211>  9
<212>  PRT
<213>  Artificial Sequence

<220>
<223>  CDRL3

<400>  14

Ser Gln Ser Thr Tyr Val Pro Leu Thr
1               5

## Description

### CROSS-REFERENCE

This application claims the priority based on Japanese Patent Application No. 2014-251898, filed in Japan on December 12, 2014, and Japanese Patent Application No. 2015-104411, filed in Japan on May 22, 2015, the entire contents of which are incorporated herein by reference in their entireties. The entire disclosures of all patents, patent applications, and literatures cited herein are also incorporated herein by reference in their entireties.

### Technical Field

The present invention relates to antibodies highly specifically recognizing amyloid β having a turn structure at amino acid positions 22 and 23, methods for measuring the amyloid β having the turn structure at amino acid positions 22 and 23 using the antibody, and methods for diagnosing and treating Alzheimer's disease.

### Background Art

Alzheimer's disease (hereinafter referred to as "AD") is generally characterized by accumulation of amyloid in senile plaques. Amyloid is composed of amyloid β protein (hereinafter referred to as "Aβ"), especially Aβ consisting of 40 or 42 amino acid residues (hereinafter referred to as "Aβ40" or "Aβ42", respectively). These proteins are generated by degradation of amyloid precursor proteins (APPs) by two proteases, β- and γ-secretases. Aβ42 has been considered to play a more important role in the development of AD as compared with Aβ40 due to its aggregation propensity and neural toxicity. Recent studies have shown that an oxidation stress affects to neurodegeneration associated with AD. Neural toxicity of Aβ42 via formation of radicals is closely associated with radicalization at tyrosine at position 10 and methionine at position 35 accompanying generation of reactive oxygen species. There is evidence that accumulation of Aβ oligomers induces AD through synaptic toxicity.

Vaccination with Aβ aggregates to AD model mice has reduced Aβ deposition in the brain and suppressed cognitive impairment. Therefore, immunization with Aβ has been believed to be a promising method for treating AD. A clinical trial immunizing AD patients with Aβ42 (AN1792) has been conducted, but dropped because of severe side effects of excessive immune activation. Subsequently, anti-Aβ antibodies have been expected to be a promising therapeutic agent for AD, and development of the antibodies has been continued up to the present.

The inventors have been studied Aβ structure utilizing systematic proline replacement and solid-state NMR, and reported a toxic conformer of Aβ having a turn structure at positions 22 and 23 of Aβ42 which is distinguished from a physiological conformer of Aβ having a turn structure at positions 25 and 26 of Aβ42. The inventors have also revealed that the toxic conformer showed strong aggregation ability and neural toxicity (Non Patent Literature 1, Patent Literature 1).

Moreover, the inventors reported that an antibody specifically recognizing the toxic conformer having a turn structure at amino acid positions 22 and 23 of Aβ42 can be generated by using a peptide antigen in which glutamic acid at position 22 of Aβ is substituted with proline to fix the turn structure at the site (Patent Literature 2).

### Citation List

### Patent Literature

Patent Literature 1: Japanese Patent Laid-Open No. 2006-265189
Patent Literature 2: WO 2011/045945

### Non Patent Literature

Non Patent Literature 1: K. Murakami, et al. (2005) J. Am. Chem. Soc., 127: 15168-15174

### Summary of Invention

The inventors have predicted that the presence of a turn structure at amino acid positions 22 and 23 of Aβ will promote amyloid aggregation and lead to the development of AD, and have investigated a possible application for diagnosis and treatment of AD by using previously reported IBL-101 antibody (deposited with the independent administrative agency, National Institute of Advanced Industrial Science and Technology, International Patent Organism Depositary (1-1-1 Tsukuba Central 6th, Higashi, Tsukuba, Ibaraki, Japan, on October 14, 2009 under the depository number: FERM BP-11290) as an antibody specifically recognizing Aβ having a turn structure at amino acid positions 22 and 23 (hereinafter referred to as "22,23-turn Aβ"). IBL-101 antibody has been particularly expected to be applied to diagnosis and treatment of AD due to its high avidity to the polymerized wild-type Aβ and its low avidity to Aβ having no turn structure. However, the results of the investigation indicated that IBL-101 antibody would not be practically applicable as a therapeutic agent despite some effects shown in the treatment test using AD model mice. The inventors further investigated causes of the problem of IBL-101 antibody.

After thorough investigation, the inventors finally focused on slight binding of IBL-101 antibody to other Aβs having no turn structure at positions 22 and 23, although IBL-101 antibody relatively specifically recognizes Aβ having a turn structure at amino acid positions 22 and 23 as well as oligomers of wild-type Aβ. The inventors generated and investigated antibodies with various specificity to 22,23-turn Aβ other than IBL-101 antibody, and found that an antibody whose specificity is higher than that of IBL-101 antibody shows excellent effects of improving cognitive function, etc. in the treatment experiment using AD model mice. Particularly, even a single dose of such an antibody showed a superior effect of improving memory of AD developed mice, which has not ever reported in previous antibodies. In addition, measurement of the amount of Aβ in cerebral spinal fluid (CSF) from AD patients and healthy subjects with using the antibody of the present invention revealed that the Aβ bound to the antibody of the present invention was abundant in the CSF from AD patients. The results of further investigation showed that the ratio of the amount of Aβ bound to the antibody of the present invention to the total amount of Aβ was higher in AD patients. The inventors further investigated the binding specificity of the antibody of the present invention in detail, and revealed that the antibody of the present invention is highly specific to 22,23-turn Aβ among Aβs having various structures such as different turn structures and linear structures, and thus shows almost no binding to Aβs having structures other than 22,23-turn Aβ. Moreover, investigation of the binding property of these antibodies to Aβ oligomers, which are believed to be most toxic in AD, revealed that the antibody of the present invention binds to oligomers of 22,23-turn Aβ much more strongly than to the monomer of 22,23-turn Aβ. From these results, the inventors found it is important to use an antibody that very specifically binds to the monomer of 22,23-turn Aβ (i.e., shows almost no binding to Aβs having other structures), and preferably further strongly binds to oligomers of 22,23-turn Aβ in the diagnosis and treatment of AD. In other words, the inventors found that an antibody highly specifically recognizing 22,23-turn Aβ or an antibody highly specifically recognizing 22,23-turn Aβ and strongly binding to oligomers of 22,23-turn Aβ can be used to treat and diagnose AD.

One reason of side effects and low effects in previous immunotherapies targeting Aβ, especially Aβ vaccine (AN1792), and previous anti-Aβ monomer antibody is thought to be the binding to physiological (nontoxic) Aβ42. Actually, Aβ monomer has been known to have advantages such as anti-inflammatory effect (Sci Transl Med. 2012 August 1; 4(145): 145ra105.) and to potentially play a physiologically important role. Thus, the anti-Aβ monomer antibody has been recognized to have adverse side effects. Although oligomers of Aβ are toxic, it has been also known that further Aβ aggregates called fibril are not toxic. Considering these, in order to target only the toxic Aβ, current antibody drugs development approaches targeting Aβ employ a strategy to utilize antibodies that bind to only Aβ oligomers but bind to neither Aβ monomer nor fibril for distinguishing toxic Aβ from physiological Aβ (Aducanumab, Crenezumab, BAN2401). In other words, the previous idea has distinguished the presence of toxicity of Aβ by the different polymerization level of Aβ rather than the structure of Aβ monomer. The inventors have previously suggested the hypothesis that, even in the same polymerization level, i.e. monomer, there should be the structure of Aβ monomers that potentially contributes toxicity of Aβ among various structures of Aβ monomers. In the present invention, the inventors have further revealed for the first time that it is important for exerting therapeutic and diagnostic effects for AD that the antibodies should "highly" specifically recognize 22,23-turn Aβ. In other words, although Aβ monomers have been understood without distinguishing its structure, the inventors found for the first time that actually the functions of Aβ monomers are very strictly regulated by its structure, that 22,23-turn Aβ is a frequently aggregated (highly toxic) Aβ while Aβs having other structures are less aggregated (low toxic, or physiological) Aβs, and that highly specifically targeting 22,23-turn Aβ is most important in the development of anti-Aβ antibody as a therapeutic or diagnostic agent for AD, which can solve the problems of side effects and low effects of previous anti-Aβ antibodies. Moreover, the antibodies of the present invention are advantageous by targeting not only Aβ oligomers but also Aβ monomers, because the antibodies of the present invention can suppress Aβ aggregation from the beginning stage in monomers before progression of Aβ aggregation (before forming Aβ oligomers), and thus are able to provide superior medicaments as compared to the antibody drugs ever developed.

Accordingly, the present invention directs to the antibodies highly specifically recognizing Aβ having a turn structure at amino acid positions 22 and 23, and the use thereof. The present invention particularly relates to the antibodies specific to 22,23-turn Aβ, which bind to 22,23-turn Aβ but do not bind to Aβs having other structures, and the use thereof. The present invention specifically relates to the inventions as described below. The term "Aβ having a turn structure at amino acid positions 22 and 23" in (1) to (31) as described below or throughout this specification may be replaced with "E22P-Aβ" or "Aβ bound by IBL-102 antibody".
(1) An antibody or immunoreactive fragment thereof that highly specifically recognizes Aβ having a turn structure at amino acid positions 22 and 23 but does not recognize Aβs having a structure other than a turn structure at amino acid positions 22 and 23.
(2) The antibody or immunoreactive fragment thereof of (1), further recognizing Aβ oligomer.
(3) The antibody or immunoreactive fragment thereof of (2), wherein the Aβ oligomer is dimer.
(4) The antibody or immunoreactive fragment thereof of any one of (1) to (3), wherein the Aβ is Aβ42.
(5) The antibody or immunoreactive fragment thereof of any one of (1) to (4), wherein the structure other than a turn structure at amino acid positions 22 and 23 of Aβ comprises at least one or more structures selected from a group consisting of: a turn structure at amino acid positions 21 and 22 of Aβ; a turn structure at amino acid positions 23 and 24 of Aβ; a turn structure at amino acid positions 24 and 25 of Aβ; a turn structure at amino acid positions 25 and 26 of Aβ; and a turn structure at amino acid positions 35 and 36 of Aβ.
(6) An antibody or immunoreactive fragment thereof that highly specifically recognizes an epitope that can be bound by an antibody having VH comprised of the amino acid sequence of SEQ ID NO: 4 and VL comprised of the amino acid sequence of SEQ ID NO: 11, but does not recognize epitopes that cannot be bound by an antibody having VH comprised of the amino acid sequence of SEQ ID NO: 4 and VL comprised of the amino acid sequence of SEQ ID NO: 11.
(7) An antibody or immunoreactive fragment thereof that competitively inhibits binding of an antibody in which VH has the amino acid sequence of SEQ ID NO: 4 and VL has the amino acid sequence of SEQ ID NO: 11 to its antigen and does not bind to an epitope not bound by an antibody in which VH has the amino acid sequence of SEQ ID NO: 4 and VL has the amino acid sequence of SEQ ID NO: 11.
(8) An antibody or immunoreactive fragment thereof wherein CDR1, CDR2, and CDR3 of heavy chain variable region have the amino acid sequences of SEQ ID NO: 5, SEQ ID NO: 6, and SEQ ID NO: 7, respectively.
(9) The antibody or immunoreactive fragment thereof of (8), wherein CDR1, CDR2, and CDR3 of light chain variable region have the amino acid sequences of SEQ ID NO: 12, SEQ ID NO: 13, and SEQ ID NO: 14, respectively.
(10) The antibody or immunoreactive fragment thereof of any one of (1) to (7), wherein the CDR1, CDR2, and CDR3 of heavy chain variable region have the amino acid sequences of SEQ ID NO: 5, SEQ ID NO: 6, and SEQ ID NO: 7, respectively.
(11) The antibody or immunoreactive fragment thereof of (10), wherein the CDR1, CDR2, and CDR3 of light chain variable region have the amino acid sequences of SEQ ID NO: 12, SEQ ID NO: 13, and SEQ ID NO: 14, respectively.
(12) An antibody or immunoreactive fragment thereof, wherein VH comprises the amino acid sequence of SEQ ID NO: 4.
(13) The antibody or immunoreactive fragment thereof of (12), wherein VL comprises the amino acid sequence of SEQ ID NO: 11.
(14) A nucleic acid molecule encoding an antibody or immunoreactive fragment thereof having the amino acid sequence of SEQ ID NO: 4.
(15) The nucleic acid molecule of (14), consisting of the nucleotide sequence from position 126 to position 479 of SEQ ID NO: 1.
(16) A nucleic acid molecule encoding an antibody or immunoreactive fragment thereof having the amino acid sequence of SEQ ID NO: 11.
(17) The nucleic acid molecule of (16), consisting of the nucleotide sequence from position 130 to position 465 of SEQ ID NO: 8.
(18) A vector capable of expressing an antibody or immunoreactive fragment thereof having the amino acid sequence of SEQ ID NO: 4, and/or an antibody or immunoreactive fragment thereof having the amino acid sequence of SEQ ID NO: 11.
(19) The vector of (18), comprising the nucleic acid molecule of (14) or (15), and/or the nucleic acid molecule of (16) or (17).
(20) A host cell comprising the vector of (18) or (19).
(21) A pharmaceutical composition comprising the antibody or immunoreactive fragment thereof of any one of (1) to (13).
(22) The pharmaceutical composition of (21), for preventing, treating, or improving AD.
(23) The pharmaceutical composition of (21), for improving cognitive function of AD patients.
(24) The pharmaceutical composition of (21), for improving memory impairment of AD patients.
(25) A diagnostic composition comprising the antibody or immunoreactive fragment thereof of any one of (1) to (13).
(26) The diagnostic composition of (25), for use in diagnosis of AD.
(27) A kit for measuring the toxic conformer of Aβ, comprising the antibody or immunoreactive fragment thereof of any one of (1) to (13).
(28) A composition for use in measurement of the toxic conformer of Aβ, comprising the antibody or immunoreactive fragment thereof of any one of (1) to (13).
(29) A method for measuring a level of the toxic conformer of Aβ in a sample, comprising a step of contacting the sample with the antibody or immunoreactive fragment thereof of any one of (1) to (13).
(30) A method for diagnosing AD, comprising steps of:
   a) contacting a sample from a subject with at least one antibody or immunoreactive fragment thereof of any one of (1) to (13) in vitro,
   b) measuring a level of an antigen bound to the antibody or immunoreactive fragment thereof, and
   c) diagnosing the presence or absence or stage of AD in the subject from the determined level of the antigen,
   wherein the subject is diagnosed as highly likely to have AD when the determined level of the antigen bound to the antibody or immunoreactive fragment thereof of any one of (1) to (13) is higher than a level of the antigen in a sample from a healthy subject.
(31) A method for diagnosing AD, comprising steps of:
   a) contacting a sample from a subject with at least one antibody or immunoreactive fragment thereof of any one of (1) to (13),
   b) measuring a level of an antigen bound to the antibody or immunoreactive fragment thereof,
   c) contacting the sample from the subject with an anti-Aβ antibody that is not specific to a structure of Aβ, and measuring the amount of the formed antibody-antigen complex, thereby determining a level of total Aβ in the sample from the subject, and
   d) calculating the ratio of the level of an antigen bound to the antibody or immunoreactive fragment thereof of any one of (1) to (13) to the level of total Aβ in the sample from the subject, and diagnosing the presence or absence or stage of AD in the subject from the calculated ratio,
   wherein the subject is diagnosed as highly likely to have AD when the ratio of the level of the antigen bound to the antibody or immunoreactive fragment thereof of any one of (1) to (13) to the level of total Aβ is higher than the ratio in a sample from a healthy subject.
(32) A method for determining the presence or absence of the toxic conformer of Aβ in a sample, comprising steps of:
   (a) contacting the sample with the antibody or immunoreactive fragment thereof of any one of (1) to (13),
   (b) detecting the toxic conformer of Aβ bound to the antibody or immunoreactive fragment thereof, and
   (c) determining the presence of the toxic conformer of Aβ in the sample when the toxic conformer of Aβ is detected in the step (b), or determining the absence of the toxic conformer of Aβ in the sample when the toxic conformer of Aβ is not detected in the step (b).
(33) A method for determining a level of the toxic conformer of Aβ in a sample, comprising steps of:
   (a) contacting the sample with the antibody or immunoreactive fragment thereof of any one of (1) to (13),
   (b) measuring the amount of the toxic conformer of Aβ bound to the antibody or immunoreactive fragment thereof, and
   (c) calculating the level of the toxic conformer of Aβ in the sample from the measured amount of the toxic conformer.

As used herein, "Aβ having a turn structure at amino acid positions 22 and 23" means Aβ having a bent (turn) structure (conformation) at amino acid positions 22 and 23 (herein referred to as "22,23-turn Aβ"). The 22,23-turn Aβ includes 22,23-turn Aβ42 and 22,23-turn Aβ40, and is preferably 22,23-turn Aβ42. "A turn structure at amino acid positions 22 and 23 of amyloid β" means a bent (turn) structure (conformation) at amino acid positions 22 and 23 of Aβ. As used herein, the turn structure at amino acid positions 22 and 23 of Aβ and/or the Aβ having this structure are collectively referred to as "the toxic conformer of Aβ". The turn structure at amino acid positions 22 and 23 of Aβ may be, for example, a bent (turn) structure at amino acid positions 22 and 23 of Aβ mutant in which glutamic acid at position 22 is substituted with proline (E22P-Aβ, such as E22P-Aβ42 or E22P-Aβ40). Since IBL-102 antibody of the present invention only recognizes Aβ having such a structure, in an alternative expression, "Aβ having a turn structure at amino acid positions 22 and 23" may be replaced with "Aβ recognized by IBL-102 antibody".

As used herein, Aβ is an abbreviation of amyloid β. Aβ42 refers to a peptide having the amino acid sequence of SEQ ID NO: 15. Aβ40 refers to a peptide having the amino acid sequence of SEQ ID NO: 16. As used herein, the positions 22 and 23 in the expression such as "at positions 22 and 23 of Aβ" or "Aβ having a turn structure at amino acid positions 22 and 23" means the amino acid positions at 22 and 23 (glutamic acid and aspartic acid in wild type, respectively) of SEQ ID NO: 15 and SEQ ID NO: 16.

The present invention relates to antibodies or immunoreactive fragments thereof highly specifically recognizing "Aβ having a turn structure at amino acid positions 22 and 23" and/or "a turn structure at amino acid positions 22 and 23 of amyloid β" (the toxic conformer of Aβ). Thus, the antibodies of the present invention highly specifically recognize 22,23-turn Aβ42 and/or 22,23-turn Aβ40.

Recognition of the toxic conformer of Aβ by the antibody or immunoreactive fragment thereof of the present invention may not depend on the type of amino acid at position 22. In other words, an "epitope" recognized by the antibody or immunoreactive fragment thereof of the present invention requires a structure and amino acids (except glutamic acid at position 22) that are present in Aβ having a turn structure at amino acid positions 22 and 23 but may not require the amino acid at position 22 to be glutamic acid. The recognition site of the Aβ toxic conformer highly specifically recognizing antibody of the present invention is not limited as long as the antibody can highly specifically recognize Aβ having a turn structure at amino acid positions 22 and 23. The site recognized by the Aβ toxic conformer highly specifically recognizing antibody of the present invention may be, for example, the amino acid at position 23 of Aβ (aspartic acid), and/or the amino acids adjacent to the amino acid at position 23, for example amino acids within 1 to 10 residues (preferably 1 to 5, 1 to 4, and 1 to 3 residues) from the amino acid at position 22 or 23 of Aβ. For example, the antibodies of the present invention may be an antibody highly specifically recognizing the toxic conformer of Aβ and recognizing the amino acid at position 23 (aspartic acid) of Aβ. Alternatively, the Aβ toxic conformer highly specifically recognizing antibody of the present invention may recognizes a conformational epitope on Aβ locating distant from positions 22 and 23, which can be generated by having the turn structure at amino acid positions 22 and 23. For example, the antibody of the present invention may recognize a region of Aβ at which Aβs form intermolecular β-sheet structure. More preferably, the antibody of the present invention recognizes asparagine at position 27 and isoleucine at position 31 of Aβ, both of which present in said region. The antibody of the present invention may bind to glutamic acid at position 3 and phenylalanine at position 4 of Aβ. The antibodies of the present invention may bind to glutamic acid at position 11, valine at position 12, histidine at position 13, and histidine at position 14 of Aβ.

Aβ aggregates to form oligomers. Further proceeded aggregation of Aβ gives protofibril, and still further proceeded aggregation of Aβ gives fibril. In the development of AD, soluble aggregates such as oligomers and protofibril are believed to exhibit some kind of toxicity. As used herein, the term "Aβ oligomers" means dimer to icosamer of Aβ or a 90 to 650 kDa aggregate of Aβ. For example, Aβ oligomer includes dimer, trimer, tetramer, pentamer, hexamer, heptamer, octamer, and nonamer, decamer of Aβ, and protofibril. In one aspect, Aβ oligomers are multimer of Aβ forming intermolecular β-sheet structure.

The antibodies of the present invention may preferably bind to an oligomer (e.g., dimer) of Aβ (preferably, 22,23-turn Aβ or E22P-Aβ42). In one aspect, the present invention relates to an antibody or immunoreactive fragment thereof that binds to both of monomer and oligomer (e.g., dimer) of 22,23-turn Aβ and/or E22P-Aβ42.

In addition to the turn structure at amino acid positions 22 and 23, Aβ is known to potentially take various structures, such as an almost linear structure and a structure having a turn structure at amino acid positions 25 and 26. As used herein, the term "other structures" (structures other than the turn structure at amino acid positions 22 and 23 of Aβ) means structures of Aβ other than the structure of Aβ having a turn structure at amino acid positions 22 and 23, and specifically can include a turn structure at amino acid positions 21 and 22 of Aβ, a turn structure at amino acid positions 23 and 24 of Aβ, a turn structure at amino acid positions 24 and 25 of Aβ, a turn structure at amino acid positions 25 and 26 of Aβ, a turn structure at amino acid positions 35 and 36 of Aβ, and linear structures (including almost linear structures, and any structure which is not included in turn structure; the same hereinafter). Alternatively, "other structures" may include a structure contained in A21P-Aβ42, E22V-Aβ42, D23P-Aβ42, V24P-Aβ42, G25P-Aβ42, G33P-Aβ42, L34P-Aβ42, M35P-Aβ42, V36P-Aβ42, G37P-Aβ42, G38P-Aβ42, V39P-Aβ42, V40P-Aβ42, and/or I41P-Aβ42, which can be, for example, the turn structure contained in these peptides. The term "Aβs having other structures" means Aβs having a structure other than a turn structure at amino acid positions 22 and 23, and specifically includes Aβ having a turn structure at amino acid positions 21 and 22, Aβ having a turn structure at amino acid positions 23 and 24, Aβ having a turn structure at amino acid positions 24 and 25, Aβ having a turn structure at amino acid positions 25 and 26, Aβ having a turn structure at amino acid positions 33 and 34, Aβ having a turn structure at amino acid positions 34 and 35, Aβ having a turn structure at amino acid positions 35 and 36, Aβ having a turn structure at amino acid positions 36 and 37, Aβ having a turn structure at amino acid positions 37 and 38, Aβ having a turn structure at amino acid positions 38 and 39, Aβ having a turn structure at amino acid positions 40 and 41, Aβ having a turn structure at amino acid positions 41 and 42, and/or Aβ having a linear structure. Alternatively, "Aβs having other structures" may include Aβ having a structure contained in A21P-Aβ42, E22V-Aβ42, D23P-Aβ42, V24P-Aβ42, G25P-Aβ42, G33P-Aβ42, L34P-Aβ42, M35P-Aβ42, V36P-Aβ42, G37P-Aβ42, G38P-Aβ42, V39P-Aβ42, V40P-Aβ42, and/or I41P-Aβ42.

In an aspect, the present invention relates to the antibody highly specifically recognizing 22,23-turn Aβ and/or a turn structure at amino acid positions 22 and 23 of Aβ. In another aspect, the present invention relates to the antibody that specifically recognize 22,23-turn Aβ and/or a turn structure at amino acid positions 22 and 23 of Aβ but do not recognize Aβs having other structures and/or other structures of Aβ. The antibody or immunoreactive fragment thereof of the present invention can specifically recognize 22,23-turn Aβ or a turn structure at amino acid positions 22 and 23 of Aβ but do not recognize Aβs having other structures and/or other structures of Aβ, for example, a turn structure at any site of Aβ other than positions 22 and 23. As used herein, the phrases "do not bind" and "do not recognize" does not necessarily mean no binding or no recognition, but means that an affinity to other structures is sufficiently low to distinguish the other structures from an antigen of interest (the toxic conformer of Aβ). For example, the phrase "do not bind" and "do not recognize" may mean that a binding intensity (e.g., fluorescence intensity) detected by ELISA or EIA for a test antibody to Aβs having other structures or to other structures of Aβ is 1/3 or less, 1/4 or less, 1/5 or less, 1/6 or less, 1/7 or less, 1/8 or less, 1/9 or less, 1/10 or less, 1/20 or less, 1/30 or less, 1/40 or less, 1/50 or less, or 1/100 or less of a binding intensity detected by the same ELISA or EIA for the test antibody to the toxic conformer of Aβ. The detection by ELISA or EIA can be performed according to the description below (about well-known measurement methods using an antibody molecule). For example, the cross-reaction rate of the antibodies of the present invention between binding to one or more peptides (including all peptides) selected from the group consisting of A21P-Aβ42, E22V-Aβ42, D23P-Aβ42, V24P-Aβ42, G25P-Aβ42, G33P-Aβ42, L34P-Aβ42, M35P-Aβ42, V36P-Aβ42, G37P-Aβ42, G38P-Aβ42, V39P-Aβ42, V40P-Aβ42, and I41P-Aβ42, and binding to main target (22,23-turnAβ or E22P-Aβ42) may be 10% or less, 8% or less, 5% or less, 4% or less, 3% or less, 2% or less, 1% or less, 0.5% or less, or 0.3% or less. Alternatively, the cross-reaction rates of the antibodies of the present invention between binding to A21P-Aβ42, E22V-Aβ42, D23P-Aβ42, V24P-Aβ42, G25P-Aβ42, and M35P-Aβ42, and the main target (22,23-turn Aβ or E22P-Aβ42) may be 0.3% or less, 0.3% or less, 0.5% or less, 0.4% or less, 0.6% or less, and 0.7% or less, respectively and further may be 0.27% or less, 0.28% or less, 0.50% or less, 0.33% or less, 0.53% or less, and 0.68% or less, respectively. The cross-reaction rate can be determined according to the method for the (Cross-Reaction Rate) as described below.

As used herein, the phrase that the antibody or immunoreactive fragment thereof "specifically" recognizes (binds to) means that an affinity of the antibody or immunoreactive fragment thereof for binding to the toxic conformer of Aβ is substantially higher than that to a peptide or protein having other amino acid sequence or other conformation. As used herein, the phrase that the antibody or immunoreactive fragment thereof "highly specifically" recognizes (binds to) means that an affinity of the antibody or immunoreactive fragment thereof for binding to their epitope or antigen is substantially higher than that to a peptide or protein having other amino acid sequence or other conformation that is very similar to the epitope or the antigen. For example, the phrase that the antibody of the present invention "highly specifically" recognizes (binds to) may mean that the antibody or immunoreactive fragment thereof binds to 22,23-turn Aβ or E22P-Aβ42 (or a turn structure at amino acid positions 22 and 23 of Aβ) with substantially higher affinity than to a peptide having the same amino acid sequence as the antigen Aβ or having an amino acid sequence similar to (1 to 2 amino acids differs from) E22P-Aβ42, or to a peptide or protein having the same amino acid sequence as antigen Aβ or having an amino acid sequence similar to (differs in 1 to 2 amino acids from) E22P-Aβ42 which has a conformation very similar to 22,23-Aβ or E22P-Aβ42 (e.g., A21P-Aβ42, D23P-Aβ42, V24P-Aβ42, G25P-Aβ42, G33P-Aβ42, L34P-Aβ42, M35P-Aβ42, V36P-Aβ42, G37P-Aβ42, G38P-Aβ42, V39P-Aβ42, V40P-Aβ42, and/or I41P-Aβ42). Herein, "bind with a substantially high affinity" means the affinity is sufficiently high to distinguish a particular amino acid sequence of interest or a particular conformation of interest from other amino acid sequences and other conformations by detection using a desired measuring device or method. For example, a substantially high affinity may mean that the binding intensity (e.g., fluorescence intensity) of a particular amino acid sequence of interest or a particular conformation of interest detected by ELISA or EIA is 3 times or more, 4 times or more, 5 times or more, 6 times or more, 7 times or more, 8 times or more, 9 times or more, 10 times or more, 20 times or more, 30 times or more, 40 times or more, 50 times or more, or 100 times or more of that of other amino acid sequences or other conformations. Accordingly, in an aspect, the present invention relates to an antibody or immunoreactive fragment thereof that highly specifically binds to E22P-Aβ42 but do not bind to one or more (preferably all) peptides selected from the group consisting of A21P-Aβ42, D23P-Aβ42, V24P-Aβ42, G25P-Aβ42, G33P-Aβ42, L34P-Aβ42, M35P-Aβ42, V36P-Aβ42, G37P-Aβ42, G38P-Aβ42, V39P-Aβ42, V40P-Aβ42, and/or I41P-Aβ42.

Also, for example, the phrase that the antibody of the present invention "highly specifically" recognizes (binds to) may mean that the antibody or immunoreactive fragment thereof does not bind to other amino acid sequences and other conformations those are very similar to the epitope or the antigen recognized by the antibody or immunoreactive fragment thereof, even under high concentration of the antibody or immunoreactive fragment thereof. For example, the phrase the antibody of the present invention "highly specifically" recognizes (binds to) may mean that the antibody or immunoreactive fragment thereof in a high concentration binds to their epitope with a substantially higher affinity than to a peptide or protein having other amino acid sequences or other conformations those are very similar to the epitope or the antigen. The high concentration of the antibody or immunoreactive fragment thereof may be, for example, 15 ng/mL or more, 30 ng/mL or more, 40 ng/mL or more, 50 ng/mL or more, 60 ng/mL or more, 70 ng/mL or more, 80 ng/mL or more, 90 ng/mL or more, 100 ng/mL or more, 110 ng/mL or more, 120 ng/mL or more, 130 ng/mL or more, 140 ng/mL or more, or 150 ng/mL or more. Specificity may be determined, for example, by an ELISA or EIA assay.

The association rate constant (Ka1) between an antibody of the present invention and the toxic conformer of Aβ or E22P-Aβ42 can be, for example, 1 x 10⁴ Ms⁻¹ or more, 1 x 10⁵ Ms⁻¹ or more, 2 x 10⁵ Ms⁻¹ or more, and 2.3 x 10⁵ Ms⁻¹ or more, and is preferably 2.32 x 10⁵ or more. The dissociation rate constant (Kd1) between an antibody of the present invention and the toxic conformer of Aβ or E22P-Aβ42 can be, for example, 1 x 10⁻³ or less, 9 x 10⁻⁴ or less, and 8 x 10⁻⁴ or less, and is preferably 7.09 x 10⁻⁴ or less. The association constant (KD) between an antibody of the present invention and the toxic conformer of Aβ or E22P-Aβ42 can be, for example, 1 x 10⁻⁸ (M) or less, 7 x 10⁻⁸ (M) or less, 5 x 10⁻⁸ (M) or less, and 4 x 10⁻⁹ or less, and is preferably 3.05 x 10⁻⁹ or less. The association rate constant (Ka1), the dissociation rate constant (Kd1), and the association constant (KD) of antibodies described herein can be measured using BIACORE (GE Healthcare Bioscience, BIACORE-X100) according to the manual provided by the manufacturer by immobilizing biotinylated E22P-Aβ42 or biotinylated wild-type Aβ42 on SA chips, then flowing a test antibody over the SA chips, measuring the association rate constant Ka1 and the dissociation rate constant Kd1, and calculating the association constant KD from the bivalent fitting.

The antibodies of the present invention may be a polyclonal or monoclonal antibody, and is preferably a monoclonal antibody. As used herein, a "monoclonal antibody" is an antibody having an almost uniform structure and reacting with a single antigenic determinant. Moreover, the antibody of the present invention includes a non-human animal antibody, an antibody having both of an amino acid sequence of a non-human animal antibody and an amino acid sequence of a human antibody, and a human antibody. The non-human animal antibody includes, for example, an antibody from mouse, rat, hamster, guinea pig, rabbit, dog, monkey, sheep, goat, chicken, duck, and the like, and is preferably an antibody from any animal that can be used to generate a hybridoma, and is more preferably an antibody from mouse, rat, or rabbit. The antibody having an amino acid sequence of a non-human animal antibody and an amino acid sequence of a human antibody includes a humanized chimeric antibody and a humanized antibody. In the above, a "chimeric antibody" is an antibody in which the constant region of a non-human animal antibody that highly specifically binds to the toxic conformer of Aβ is genetically modified to the same constant region as a human antibody, and is preferably a human-mouse chimeric antibody (see European Patent Publication No. EP0125023A). A "humanized antibody" refers to an antibody in which the primary structure except for the complementarity determining regions (CDRs) of H chain and L chain of a non-human animal antibody highly specifically binding to the toxic conformer of Aβ is genetically modified to a corresponding primary structure of a human antibody. The CDR may be defined by the method described by either Kabat, et al. ("Sequences of Proteins of Immunological Interest", Kabat, E. et al., U.S. Department of Health and Human Services, 1983) or Chothia, et al. (Chothia & Lesk (1987) J. Mol. Biol., 196: 901-917). A "human antibody" refers to a human antibody that is an expression product of antibody gene completely derived from human, and includes, for example, a monoclonal antibody produced by a transgenic animal transduced with a human gene involved in antibody production (see, European Patent Publication EP0546073A). For example, when the antibody of the present invention is used for treatment, prevention, or diagnosis which includes administration of the antibody to a living body, the antibody of the present invention is preferably a human/non-human animal chimeric antibody, a humanized antibody, or a human antibody.

The immunoglobulin class of the antibody of the present invention is not particularly limited and may be any immunoglobulin class (isotype) of IgG, IgM, IgA, IgE, IgD, or IgY, and preferably IgG. When the antibody of the present invention is IgG, it may be any subclass (IgG1, IgG2, IgG3, or IgG4). The antibodies of the present invention may be monospecific, bispecific, or trispecific (e.g., WO1991/003493).

It is known that variable regions (especially CDRs) of antibody are responsible to its binding properties, and it is well known to those skilled in the art that a binding properties of an antibody is retained even in an antibody fragment, incomplete antibody. As used herein, an "immunoreactive fragment" means a protein or peptide comprising a portion (partial fragment) of an antibody which retains the effect (immunoreactivity, binding property) of the antibody on the antigen. Such immunoreactive fragment includes, for example, F(ab')₂, Fab', Fab, Fab₃, a single-stranded Fv (hereinafter referred to as "scFv"), a (tandem) bispecific single-stranded Fv (sc(Fv)₂), a single-stranded triplebody, a nanobody, a divalent V_{H}H, a pentavalent V_{H}H, a minibody, a (double-stranded) diabody, a tandem diabody, a bispecific tribody, a bispecific bibody, a dual affinity retargeting (DART) molecule, a triabody (or tribody), a tetrabody (or [sc(Fv)₂]₂), or a (scFv-SA)₄) disulfide-bonded Fv (hereinafter referred to as "dsFv"), a compact IgG, a heavy-chain antibody, or polymers thereof (see, Nature Biotechnology, 29 (1): 5-6 (2011); Maneesh Jain, et al., TRENDS in Biotechnology, 25 (7) (2007): 307-316; and Christoph stein, et al., Antibodies (1): 88-123 (2012)). In this specification, the immunoreactive fragment may be any of monospecific, bispecific, trispecific, and multispecific fragment.

Specific examples of the antibody or immunoreactive fragment thereof which highly specifically recognizes the toxic conformer of Aβ as described herein includes IBL-102 antibody or its derivative. For example, the antibody of the present invention includes (i) an antibody or immunoreactive fragment thereof, wherein CDR1, CDR2, and CDR3 of heavy chain variable region have the amino acid sequences of SEQ ID NOS: 5, 6, and 7, respectively; and (ii) the antibody or immunoreactive fragment thereof of (i), wherein CDR1, CDR2, and CDR3 of light chain variable region have the amino acid sequences of SEQ ID NOS: 12, 13, and 14, respectively. Alternatively, "the antibody or immunoreactive fragment thereof which specifically recognizes a turn structure at amino acid positions 22 and 23 of Aβ" as described herein includes, for example, an antibody or immunoreactive fragment thereof selected from the group consisting of the following (iii) to (viii): (iii) an antibody or immunoreactive fragment thereof, wherein VH has an amino acid sequence encoded by a nucleic acid sequence which hybridizes with a nucleic acid sequence encoding the amino acid sequence of SEQ ID NO: 4 under a stringent condition, and which highly specifically recognizes a turn structure at amino acid positions 22 and 23 of Aβ but does not recognize any turn structures at other sites of Aβ, (iv) the antibody or immunoreactive fragment thereof of (iii), wherein VL has an amino acid sequence encoded by a nucleic acid sequence which hybridizes with a nucleic acid sequence encoding the amino acid sequence of SEQ ID NO: 11 under a stringent condition; (v) an antibody or immunoreactive fragment thereof, wherein VH has an amino acid sequence having 80% or more identity to the amino acid sequence of SEQ ID NO: 4, and which highly specifically recognizes a turn structure at amino acid positions 22 and 23 of Aβ but does not recognize any turn structure at other sites of Aβ; (vi) the antibody or immunoreactive fragment thereof of (v), wherein VL has an amino acid sequence having 80% or more identity to the amino acid sequence of SEQ ID NO: 11; (vii) an antibody or immunoreactive fragment thereof, wherein VH has the amino acid sequence of SEQ ID NO: 4; (viii) the antibody or immunoreactive fragment thereof of (vii), wherein VL having the amino acid sequence of SEQ ID NO: 11. The antibody or immunoreactive fragment thereof of (iii) to (vi) may further includes CDR1, CDR2, and CDR3 of heavy chain variable region having the amino acid sequences of SEQ ID NOS: 5, 6, and 7, respectively, and/or CDR1, CDR2, and CDR3 of light chain variable region having the amino acid sequences of SEQ ID NOS: 12, 13, and 14, respectively. In this specification, the reference to "IBL-102 monoclonal antibody" may include the antibody and immunoreactive fragment thereof of above (i) to (viii) in its broad meaning (especially except for the case in which such an interpretation is inconsistent).

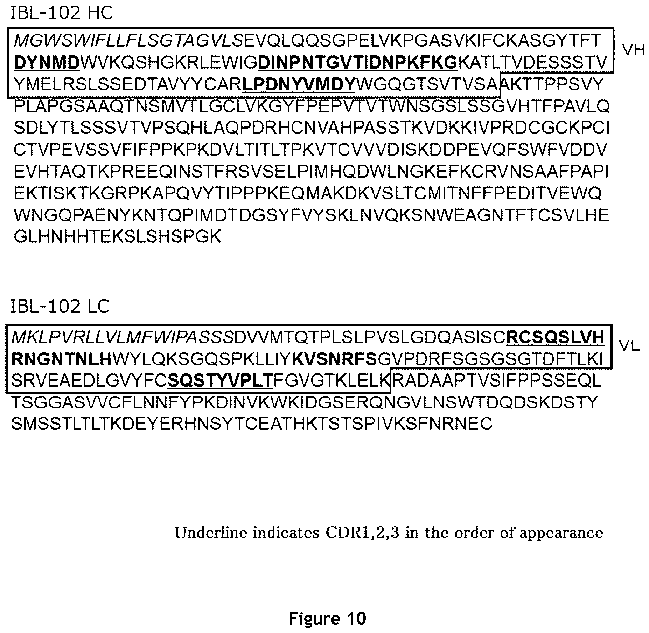

In this specification, "the amino acid sequences of SEQ ID NO: 5, SEQ ID NO: 6, and SEQ ID NO: 7" may be replaced with the three underlined and bolded amino acid sequences in the amino acid sequence of IBL-102HC of Fig.10, respectively in the order of appearance leftward from upper left. The amino acid sequences may be SEQ ID NO: 5 (DYNMD), SEQ ID NO: 6 (DINPNTGVTIDNPKFKG), and SEQ ID NO: 7 (LPDNYVMDY). Herein, "the amino acid sequences of SEQ ID NO: 12, SEQ ID NO: 13, and SEQ ID NO: 14" may be replaced with the three underlined and bolded amino acid sequences in the amino acid sequence of IBL-102LC in Fig.10, respectively in the order of appearance leftward from upper left. The amino acid sequences may be SEQ ID NO: 12 (RCSQSLVHRNGNTNLH), SEQ ID NO: 13 (KVSNRFS), and SEQ ID NO: 14 (SQSTYVPLT). The term "the amino acid sequence of SEQ ID NO: 4" may be replaced with the amino acid sequence in the box shown as IBL-102HC in Fig.10. The term "the amino acid sequence of SEQ ID NO: 11" may be also replaced with the amino acid sequence in the box shown as IBL-102LC in Fig.10.

IBL-102 monoclonal antibody can bind to a substance closely correlated with AD and can exert therapeutic effect, which indicates that an antigen bound by IBL-102 monoclonal antibody (or a protein having an epitope bound by IBL-102 monoclonal antibody) is deeply involved in development and progression of AD and toxicity of Aβ. Such efficacy was not confirmed for IBL-101 antibody, which was obtained by using an antigen having a turn structure at amino acid positions 22 and 23 of Aβ. Thus, antibodies or immunoreactive fragments thereof that bind to the antigen (or the epitope) which is bound by IBL-102 monoclonal antibody with the almost same specificity as IBL-102 are considered to be used in the diagnosis and treatment of AD in the same manner as IBL-102 monoclonal antibody. Accordingly, in another aspect, the present invention relates to antibodies or immunoreactive fragments thereof highly specifically binding to the antigen (or the epitope) bound by IBL-102 monoclonal antibody. For example, the present invention may be an antibody or immunoreactive fragment thereof that highly specifically recognizes the epitope present in Aβ which is bound by IBL-102 antibody, but that does not recognize any epitope present in Aβ which is not bound by IBL-102 antibody. Alternatively, the present invention may be an antibody or immunoreactive fragment thereof that highly specifically recognizes Aβ that is bound by IBL-102 antibody (or E22P-Aβ42), but does not recognize Aβs having other structures that are not bound by IBL-102 antibody. In another aspect, the present invention relates to an antibody or immunoreactive fragment thereof that competes with IBL-102 antibody in specific binding to Aβ (e.g., 22,23-turn Aβ, E22P-Aβ42, or Aβ oligomers (including an oligomer of E22P-Aβ42)), which preferably does not bind to Aβ having structures that are not bound by IBL-102 antibody.

Whether a test antibody highly specifically recognizes (binds to) an epitope or an antigen of interest can be determined by measuring the binding of the antibody to a peptide having the epitope or the antigen as well as by measuring the binding of the antibody to other epitopes, an antigen having other structures, or other antigens. The binding can be measured according to the following description (for known measurement methods using an antibody molecule). Whether a test antibody competes with IBL-102 antibody in specific binding to Aβ (e.g., a monomer or oligomer of 22,23-turn Aβ, or E22P-Aβ42) can be determined by contacting IBL-102 antibody alone or in combination with a test antibody to an antigen (e.g., a monomer or oligomer of 22,23-turn Aβ, or E22P-Aβ42), and then comparing the amounts of IBL-102 antibody bound to the antigen. When the amount of IBL-102 antibody bound to the antigen is less in combination with the test antibody than in using IBL-102 antibody alone, the test antibody can be determined as competing with IBL-102 antibody. The binding of the antibody to the antigen can be measured according to the description below (for known measurement methods using an antibody molecule).

An antibody or immunoreactive fragment thereof that highly specifically binds to the antigen (or the epitope) bound by IBL-102 monoclonal antibody competes with IBL-102 antibody in specific binding to Aβ (or E22P-Aβ42), and does not bind to Aβ having a structure which are not bound by IBL-102 antibody. Preferably, the antibody or immunoreactive fragment thereof that highly specifically binds to the antigen (or the epitope) bound by IBL-102 monoclonal antibody further binds to an Aβ oligomer (including E22P-Aβ42 oligomer).

### (Nucleic Acid Molecule, Vector, and Host Cell)

In another aspect, the present invention relates to a nucleic acid molecule having a polynucleotide encoding the above antibody of the present invention. Specifically, the nucleic acid molecule of the present invention comprises a nucleic acid molecule having a polynucleotide encoding the amino acid sequence of SEQ ID NO: 4 for VH, having a polynucleotide encoding the amino acid sequence of SEQ ID NO: 11 for VL, or having a polynucleotide encoding the amino acids of SEQ ID NO: 4 for VH and a polynucleotide encoding the amino acid sequence of SEQ ID NO: 11 for VL. The polynucleotide encoding the amino acids of SEQ ID NO: 4 for VH may be, for example, a polynucleotide having the base sequence from position 126 to position 479 of SEQ ID NO: 1. The polynucleotide encoding the amino acid sequence of SEQ ID NO: 11 for VL may be a polynucleotide having the base sequence from position 130 to position 465 of SEQ ID NO: 8. The present invention further comprises a vector having the above nucleic acid molecule. The vector is not particularly limited as long as it can be used for antibody expression, and suitable plasmid vectors and the like can be selected according to the host to be used. In another aspect, the present invention relates to a host cell including the vector. The host cell is not particularly limited as long as it can be used for antibody expression, and includes a mammalian cell (mouse cell, rat cell, rabbit cell, human cell, etc.), yeast, and microorganism (such as Escherichia coli).

### (Pharmaceutical Composition)

In another aspect, the present invention relates to pharmaceutical compositions comprising the above described antibody or immunoreactive fragment thereof of the present invention as described above as an active ingredient. Diseases targeted by the pharmaceutical compositions of the present invention can include AD. Thus, the pharmaceutical compositions of the present invention can be an agent for preventing, treating, suppressing the progression of, or improving AD. The pharmaceutical compositions of the present invention can be for improving cognitive impairment (short-term or long-term memory impairment, disorientation, learning disability, impaired attention, spatial cognitive function, and/or impaired ability to solve problems) in AD patients. The present invention also relates to a use of an antibody or immunoreactive fragment thereof of the present invention for manufacturing said pharmaceutical compositions.

The pharmaceutical compositions of the present invention may be in any formulation, regardless of whether they are administered orally or parenterally, as long as they are formulations that can be administered to patients. Compositions for parenteral administration can include, for example, an injection, a nasal drop, a suppository, a poultice, an ointment, and the like, and is preferably an injection. Dosage forms of the pharmaceutical compositions of the present invention can include, for example, a liquid or a lyophilized formulation. In using the pharmaceutical composition as an injection, additives may optionally be contained that include a dissolution aid such as propylene glycol and ethylenediamine; a buffer such as phosphate; a tonicity agent such as sodium chloride and glycerin; a stabilizer such as sulfite; a preservative such as phenol; and a soothing agent such as lidocaine (see "IYAKUHIN TENKABUTSU JITEN" YAKUJI NIPPO LIMITED, "Handbook of Pharmaceutical Excipients Fifth Edition" APhA Publications). In using the pharmaceutical compositions as an injection, vessels for storing the pharmaceutical compositions of the present invention can include an ampule, a vial, a prefilled syringe, a cartridge for pen-shaped syringe, a bag for injection, and the like.

### (Kit)

In another aspect, the present invention also relates to a kit comprising an antibody or immunologically binding fragment thereof as described above. In an aspect, the kit of the present invention may be a kit for detecting or measuring an antigen bound by IBL-102 antibody. In another aspect, the kit for measurement of the present invention may be a kit for detecting or measuring the toxic conformer of Aβ. In further another aspect, the kit of the present invention can be used for the diagnosis of AD. The present invention includes use of antibodies or immunoreactive fragments thereof of the present invention as described above for manufacturing such a kit.

The kit of the present invention preferably comprise a carrier selected from the group consisting of a solid phase, a hapten, and an insoluble carrier. The kit for measurement can be based on a known detection and/or measurement method using an antibody molecule. As used herein, "a known detection and/or measurement method using an antibody molecule" includes, for example, a kit for labeling immunoassay including a kit for enzyme immunoassay (EIA), a kit for simple EIA, a kit for enzyme-linked immunosorbent assay (ELISA), a kit for radioimmunoassay (RIA), and a kit for fluorescence immunoassay (FIA); a kit for immunoblotting including a kit for Western blotting; a kit for immunochromatography including a kit for gold colloid aggregation; a kit for chromatography including a kit for ion exchange chromatography and a kit for affinity chromatography; a kit for turbidimetric immunoassay (TIA); a kit for nephelometric immunoassay (NIA); a kit for colorimetry; a kit for latex agglutination immunoassay (LIA); a kit for particle counting immunoassay (CIA); a kit for chemiluminescent immunoassay (CLIA, CLEIA); a kit for precipitation reaction; a kit for surface plasmon resonance (SPR); a kit for resonant mirror detector (RMD); a kit for comparative interferometry, and the like. Whether a kit can be used for a desired detection and/or measurement can be confirmed by performing each measurement method using the standard sample or samples of interest in the manner well known to those skilled in the art, and then determining whether the kit is able to be used for the aimed detection and/or measurement.

For example, the kit of the present invention may be a kit for immunochemical measurement, comprising (i) the first antibody that is an antibody or immunoreactive fragment thereof of the present invention immobilized onto a solid phase or hapten and (ii) the second antibody that is a labeled anti-Aβ antibody (including anti-Aβ42 antibody and anti-Aβ40 antibody; the same shall apply throughout this specification). When the kit of the present invention comprises a hapten, the kit may further comprise a substance immobilized onto a solid phase that specifically binds to the hapten. Alternatively, the first antibody may be a labeled antibody, and the second antibody may be an immobilized antibody.

Alternatively, the kit of the present invention may be a kit for immunochemical measurement, comprising (i) a first antibody that is an antibody or immunoreactive fragment thereof of the present invention immobilized onto a solid phase and (ii) a second antibody that is an anti-Aβ antibody immobilized onto a hapten. The kit may further comprise a labeled substance specifically binding to the hapten. Alternatively, the first antibody may be a hapten-binding antibody, and the second antibody may be a solid phase-immobilized antibody.

Alternatively, the kit of the present invention may be a kit for immunochemical measurement, comprising (i) the first antibody that is an antibody or immunoreactive fragment thereof of the present invention immobilized onto an insoluble carrier and (ii) the second antibody that is an anti-Aβ antibody immobilized onto an insoluble carrier.

In the examples of any of the kit as described above, each of the first antibody and the second antibody recognizes a different site of Aβ (including Aβ42 and Aβ40; the same shall apply throughout this specification). Also, the first antibody may be a monoclonal antibody or immunoreactive fragment thereof, and the second antibody may be a polyclonal antibody or immunoreactive fragment thereof. The first antibody may be a polyclonal antibody or immunoreactive fragment thereof, and the second antibody may be a monoclonal antibody or immunoreactive fragment thereof. Alternatively, both the first antibody and the second antibody may be a monoclonal antibody or immunoreactive fragment thereof. In the kit as described herein, for example, the first antibody may be an antibody of the present invention as described above, and the second antibody may be an antibody specifically recognizing a region adjacent to the N-terminus (a region from 1 to 20 amino acids from the N-terminus) or a region adjacent to the C-terminus (a region from 1 to 20 amino acids from the C-terminus) of Aβ.

When the kit of the present invention comprises an antibody or immunobinding fragment thereof with a label, the label can include detectable labels such as a radioactive label, an enzyme, a fluorescent label, a bioluminescent label, a chemiluminescent label, a metal. Examples of such labels can include, but are not limited to, detectable labels including a radioactive label such as ³²P, ³H, ¹²⁵I, and ¹⁴C; enzymes such as β-galactosidase, peroxidase, alkaline phosphatase, glucose oxidase, lactate oxidase, alcohol oxidase, monoamine oxidase, and horseradish peroxidase; coenzymes or prosthetic groups such as FAD, FMN, ATP, biotin, and heme; fluorescent labels such as a fluorescein derivative (such as fluorescein isothiocyanate (FITC) and fluorescein thioflubamyl), a rhodamine derivative (such as tetramethyl rhodamine, trimethyl rhodamine (RITC), Texas Red, and rhodamine 110), a Cy dye (Cy3, Cy5, Cy5.5, Cy7), Cy-chrome, Spectrum Green, Spectrum Orange, propidium iodide, allophycocyanin (APC), R-phycoerythrin (R-PE); bioluminescent labels such as luciferase; luminol derivatives such as luminol, isoluminol, N-(4-aminobutyl)-N-ethylisoluminose ester; acridinium derivatives such as N-methyl acridinium ester, N-methyl acridinium acylsulfonamide ester; chemiluminescent labels such as lucigenin, adamantyl dioxetane, an indoxyl derivative, a ruthenium complex; metals such as gold colloid, and the like.

The kit of the present invention may optionally comprise a coloring reagent, a reagent for stopping a reaction, a standard antigen reagent, a reagent for pretreating samples, a blocking reagent, and the like. When the kit of the present invention comprise an antibody with a label, the kit may further comprise a substrate reacting with the label. The kit of the present invention may further comprise a package containing components of the kit, such as a paper box or plastic case, an instruction manual, and the like.

Samples that can be used for the kit of the present invention include, for example, a tissue specimen or fluid specimen collected from a subject for biopsy, and biopsies to be used are not particularly limited as long as they can be subject to a desired detection and/or measurement, and biopsies can include, for example, blood, plasma, serum, lymphatic fluid, urine, serosity, spinal fluid, cerebral spinal fluid, synovial fluid, aqueous humor, tear, saliva, brain tissue, or fractions or processing products thereof. Analyses by the kit of the present invention can performed qualitatively, quantitatively, or semiquantitatively.

### (Agents for Detection, Measurement, or Diagnosis and Compositions for Detection, Measurement, or Diagnosis)

In another aspect, the present invention relates to agents or compositions for use in detection and/or measurement of an antigen bound by IBL-102 antibody or the toxic conformer of Aβ, the agents or compositions comprising an antibody or immunoreactive fragment thereof of the present invention (hereinafter referred to as "agents or compositions for measurement"). In this specification, agents or compositions for measurement include an agent or composition used for the diagnosis of AD, namely, a diagnostic agent for AD or a diagnostic composition for AD. Alternatively, the present invention relates to a use of an antibody or immunoreactive fragment thereof of the present invention for manufacturing the diagnostic composition.

For example, when intended for use outside a living body (in vitro or ex vivo), agents or compositions for detection and/or measurement of the present invention may optionally have composition similar to those in the kit of the present invention as described above.

Agents or compositions for detection and/or measurement of the present invention may be in a form for administration to a living body (hereinafter referred to as "agents or compositions for detection and/or measurement in vivo"), including a form for diagnostic imaging. The antibodies or immunoreactive fragments thereof may comprise a labeling agent or may be bound to a labeling agent when contained in the agents or compositions for detection and/or measurement in vivo. The labeling agent includes, for example, an agent well known to those skilled in the art, such as a radioactive isotope, and is preferably a positron emitting radioactive isotope or a gamma emitting radioactive isotope, and includes, but is not limited to, ¹³¹I, ¹²³I, ¹²⁴I, ⁸⁶Y, ⁶²Cu, ⁶⁴Cu, ¹¹¹In, ⁶⁷Ga, ⁶⁸Ga, ^{99m}Tc, ^{94m}Tc, ¹⁸F, ¹¹C, ¹³N, ¹⁵O, and ⁷⁵Br. In using as the agents or being contained in compositions for detection and/or measurement in vivo, the antibody or immunoreactive fragment thereof is preferably a chimeric antibody, humanized antibody, or human antibody. The agents or compositions for detection and/or measurement in vivo can be in a pharmaceutically acceptable form suitable for administration to human and may comprise a physiologically acceptable additive agent, for example, a pharmaceutically acceptable diluent, a buffering agent, a solubilizing agent, a soothing agent, a solvent, a stabilizing agent, or an antioxidant. Dosages of agents or compositions for detection and/or measurement in vivo of the present invention can be appropriately selected depending on a target site; a detecting, measuring, or diagnosing method to be used; age, sex, and other conditions of a subject; and the extent of disease.

As used herein, "XNY" (wherein X and Y represent a one-letter code of amino acids; and N is a natural number) means that the amino acid X at position N is substituted with the amino acid Y. Herein, "Aβp-q" (wherein p and q are each a natural number) means a peptide consisting of amino acids from position p to position q of Aβ. Particularly, Aβ1-40 and Aβ1-42 are referred to as Aβ40 and Aβ42, respectively. For example, E22P-Aβ42 represents a peptide consisting of amino acids from position 1 to position 42 of Aβ in which glutamic acid at position 22 is substituted with proline. Also, G9C,E22P-Aβ9-35 represents a peptide consisting of amino acids from position 9 to position 35 of Aβ in which glycine at position 9 is substituted with cysteine and glutamic acid at position 22 is substituted with proline.

### Advantageous Effects of Invention

The antibodies of the present invention can bind to the toxic conformer of Aβ with extremely high specificity and thus can have beneficial effects in various applications targeting toxic conformers of Aβ. For example, the antibodies of the present invention can be used for diagnosis of AD. The antibodies of the present invention can also bind to both monomer and dimer of the toxic conformer of Aβ and thus can be used for the treatment or prevention of a disease the development or exacerbation of which is affected by polymerization of Aβ, for example, AD.

### Brief Description of Drawings

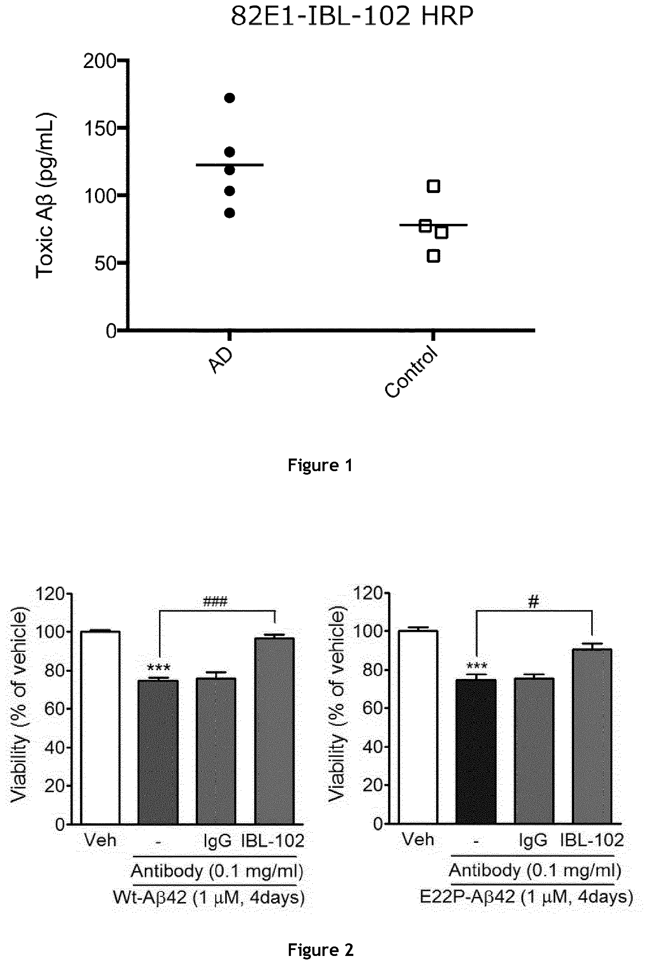

[Figure 1] Figure 1 is a graph showing a comparison of the amounts of Aβ bound to IBL-102 antibody in cerebral spinal fluids from Alzheimer's disease patients (AD) and healthy subjects (Control). The vertical axis shows the amount (pg/mL) of the toxic conformer of Aβ (Aβ bound to IBL-102 antibody).
[Figure 2] Figure 2 is a graph showing results investigated for a protecting effect of IBL-102 antibody on neural toxicity induced by Aβ42 using rat primary neurons. The left graph shows the results in the wild-type Aβ42 (Wt-Aβ42)-added group, and the right graph shows the results in the E22P-Aβ42-added group. The vertical axis shows the mean viability (%) relative to the group of no cytotoxic stimulation and no antibody addition (Veh). The error bars show standard error. From the left to right, in each graph, the bars show the result of no cytotoxic stimulation and no antibody addition (Veh), the result of cytotoxic stimulation with no antibody addition, the result of cytotoxic stimulation with IgG addition (IgG), and the result of cytotoxic stimulation with IBL-102 addition (IBL-102). *** shows p < 0.001 compared with veh, # shows p < 0.05, and ### shows p < 0.001.

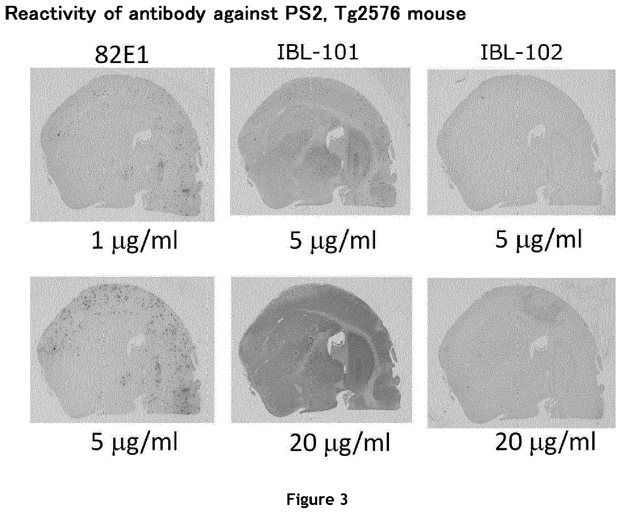

[Figure 3] Figure 3 shows images of immunohistochemically stained brain sections of AD model mice to investigate an ability of 82E1, IBL-101, and IBL-102 to stain senile plaques. The numerical value below each photograph shows each concentration of the antibody used for immunostaining.

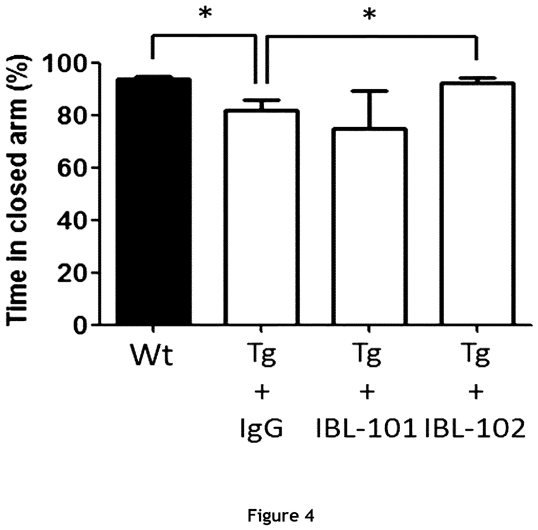

[Figure 4] Figure 4 shows the results of the elevated plus-maze test to evaluate suppressing effect of IBL-102 antibody on neurologic symptoms exhibited in AD model mice. The vertical axis shows the mean rate (%) of the duration of time staying in the closed arm, and the error bars show standard error. From the left to right, in the graph, the bars show the results of wild-type mice with no antibody administration (Wt), AD model mice with IgG administration (Tg + IgG), AD model mice with IBL-101 administration (Tg + IBL-101), and AD model mice with IBL-102 administration (Tg + IBL-102). * shows p < 0.05.

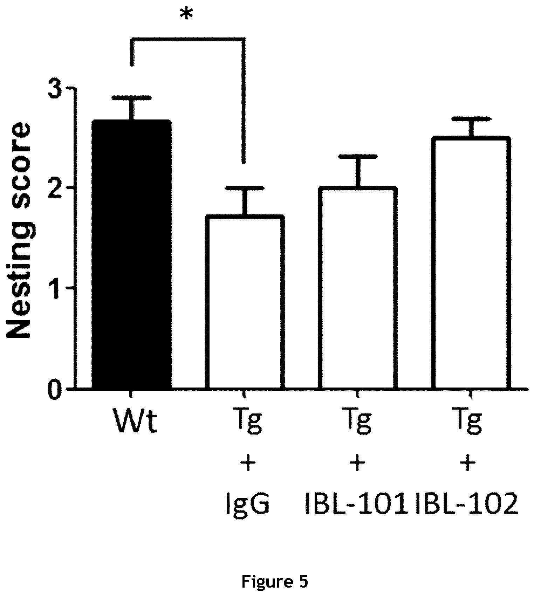

[Figure 5] Figure 5 is a graph showing suppressing effect of IBL-102 antibody on neurologic symptom exhibited in AD model mice determined by the Nesting test. The vertical axis shows the mean score, and the error bars show standard error. From the left to right, in the graph, shown are the results of wild-type mice with no antibody administration (Wt), AD model mice with IgG administration (Tg + IgG), AD model mice with IBL-101 administration (Tg + IBL-101), and AD model mice with IBL-102 administration (Tg + IBL-102). * shows p < 0.05.

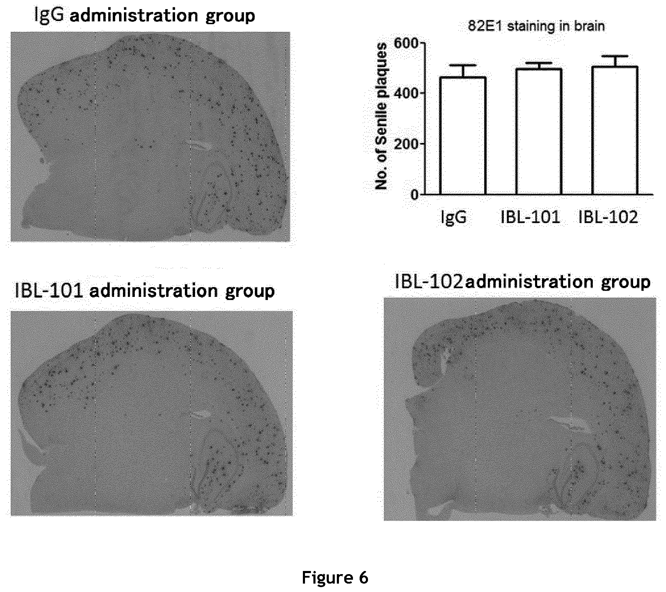

[Figure 6] Figure 6 shows images of immunohistochemically stained senile plaques in the brain of AD model mice and shows a graph showing the counts of stained senile plaques to investigate an ability of IBL-102 antibody to suppress senile plaque accumulation in the brain of AD model mice. The vertical axis of the graph shows the average count of senile plaques stained by 82E1 antibody, and the error bars show standard error. The horizontal axis shows the administered antibodies.

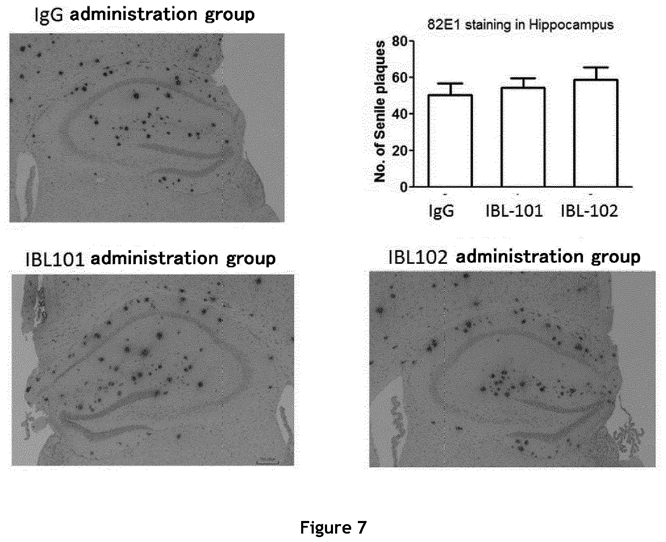

[Figure 7] Figure 7 shows images of immunohistochemically stained senile plaques in the hippocampus of AD model mice and shows a graph showing the counts of stained senile plaques to investigate an ability of IBL-102 antibody to suppress senile plaque accumulation in the hippocampus of AD model mice. The vertical axis of the graph shows the average count of senile plaques stained by 82E1 antibody, and the error bars show standard error. The horizontal axis shows the administered antibodies.

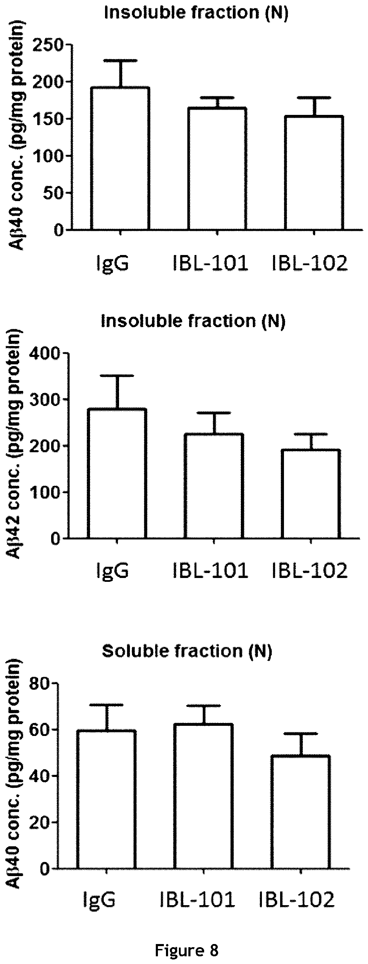

[Figure 8] Figure 8 shows graphs showing suppressing effects of IBL-102 antibody on Aβ accumulation in the brain of AD model mice evaluated by ELISA. Each vertical axis of the graphs shows, From the top to the bottom, the average concentration of Aβ40 in the insoluble fraction of the brain, the average concentration of Aβ42 in the insoluble fraction of the brain, and the average concentration of Aβ40 in the soluble fraction of the brain (all, in pg/mg protein), and the error bars show standard error. The horizontal axis shows the administered antibodies.

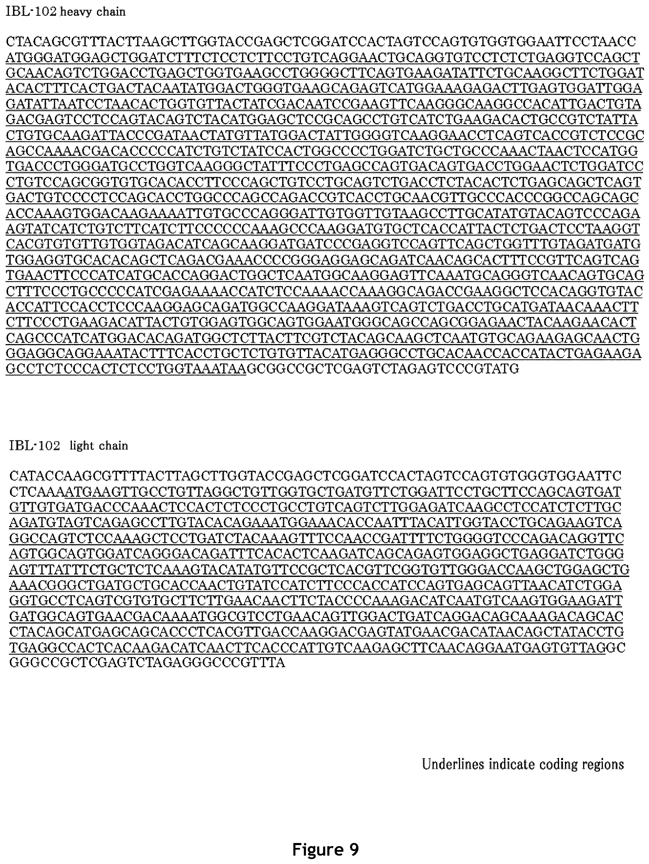

[Figure 9] Figure 9 shows the base sequence of IBL-102 antibody (wherein the base sequences of heavy chain and light chain are set forth in SEQ ID NO: 1 and SEQ ID NO: 8, respectively).
[Figure 10] Figure 10 shows the amino acid sequences of IBL-102 antibody. The amino acid sequences of heavy chain (HC) and light chain (LC) are set forth in SEQ ID NO: 2 and SEQ ID NO: 9, respectively. The sequences with oblique line represent signal sequences, the underlined sequences represent CDRs, and the sequences in the box represent variable regions.

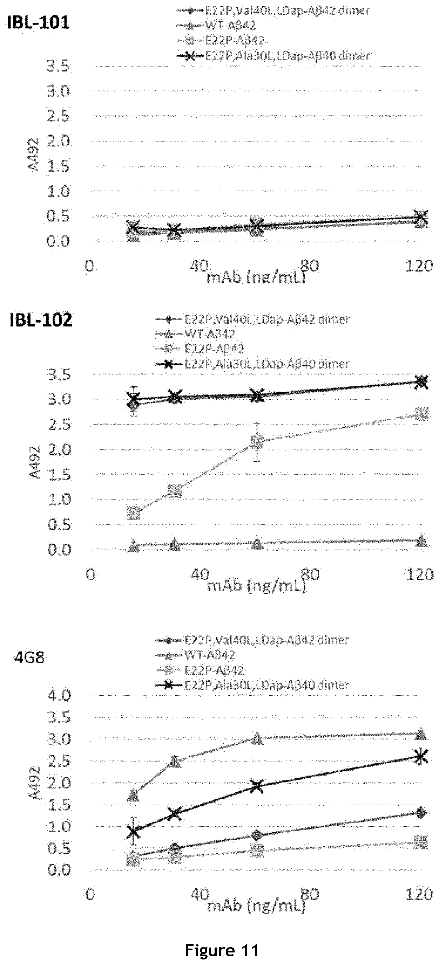

[Figure 11] Figure 11 is a graph showing binding capacity to Aβ polymer. The vertical axis shows the absorbance at 492 nm, and the horizontal axis shows the concentration (ng/mL) of test antibodies. Each graph shows, from the top to the bottom, the results of IBL-101, IBL-102, and 4G8 used as a test antibody.

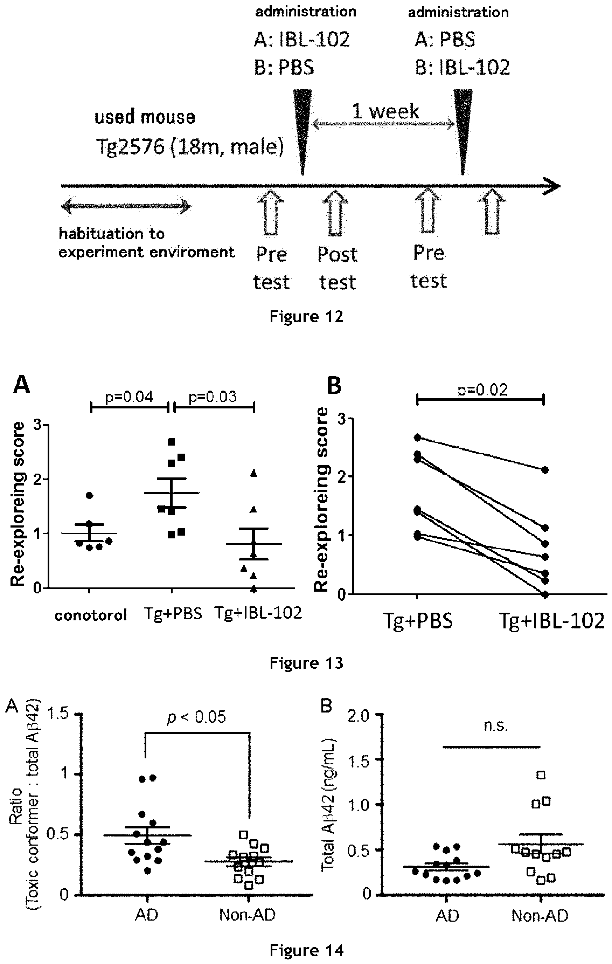

[Figure 12] Figure 12 is a diagram showing the protocol of single administration of IBL-102.
[Figure 13] Figure 13 represents graphs showing test results of single administration of IBL-102. A shows re-exploring scores of control group, Tg2576 mice received PBS, and Tg2576 mice received IBL-102. B shows comparison of re-exploring scores of each individual of Tg2576 mice on receiving PBS or IBL-102 antibody. The vertical axis shows re-exploring score.
[Figure 14] Figure 14 represents graphs showing total Aβ42 (right) and the ratio of Aβ bound to IBL-102 antibody to total Aβ42 (left) in cerebral spinal fluids from Alzheimer's disease patients (AD patient group) and from non-AD control group (non-AD control group). The vertical axis shows Aβ42 (ng/mL) (right) or the ratio of the toxic conformer of Aβ (Aβ bound to IBL-102 antibody) to total Aβ (left).

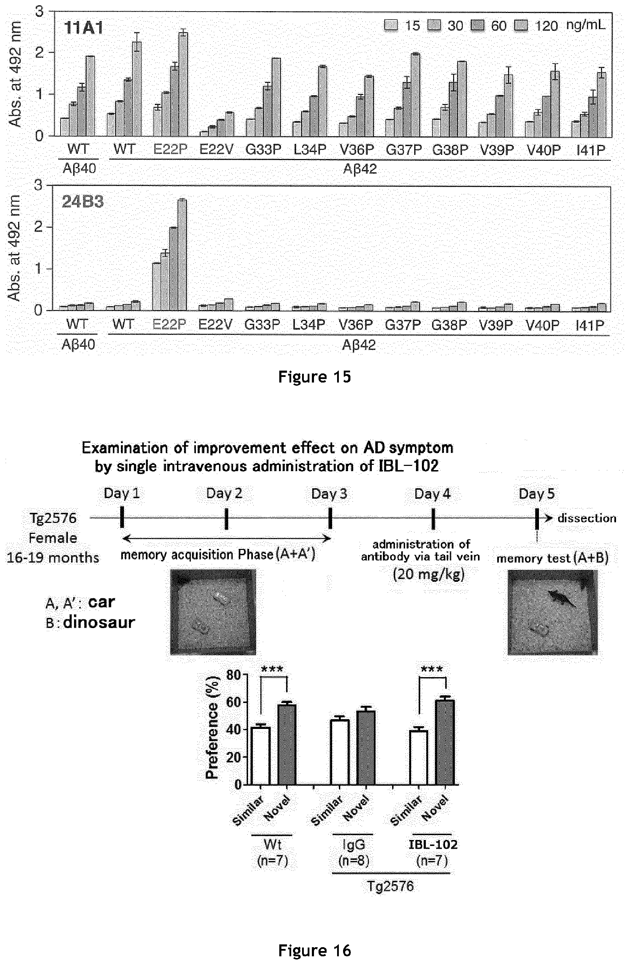

[Figure 15] Figure 15 represents graphs showing the results of an enzyme immunoassay for binding capacity of IBL-102 antibody and IBL-101 antibody to each antigen. The upper graph shows the results of IBL-101 used as an antibody, and the lower graph shows the results of IBL-102 used as an antibody. In both of the graphs, the horizontal axis show the antigens used, from the left to the right, wild-type Aβ40, wild-type Aβ42, E22P-Aβ42, E22V-Aβ42, G33P-Aβ42, L34P-Aβ42, V36P-Aβ42, G37P-Aβ42, G38P-Aβ42, V39P-Aβ42, V40P-Aβ42, and I41P-Aβ42. The vertical axis shows the absorbance at 492 nm. Four bars shown for each antigen shows, from the left to the right, results for 15 ng/mL, 30 ng/mL, 60 ng/mL, and 120 ng/mL of the antibody.
[Figure 16] Figure 16 shows the schedule and results of single administration test. The vertical axis shows (the number of contact with a novel object B) / ((the number of contact with a known object A) + (the number of contact with the novel object B)) (Novel), or (the number of contact with the known object A) / ((the number of contact with the known object A) + (the number of contact with the novel object B)) (Similar). "Wt" shows wild-type mice, "IgG" shows Tg2576 mice received IgG, "IBL-102" shows Tg2576 mice received IBL-102. "n" is the number of mice used in each group. *** shows P < 0.0005.

### Description of Embodiments

### 1. Production of antibody

### (Acquisition of antibody)

The antibodies of the present invention can be produced by immunizing non-human mammals or birds with a substance having a turn structure at amino acid positions 22 and 23 of Aβ42, preferably E22P-Aβ42, Aβ-lactam (22K-23E), or P3-Aβ42 (Japanese Patent Laid-Open No. 2006-265189; K. Murakami, et al. (2005) J. Am. Chem. Soc., 127: 15168-15174) as an immunogen, optionally in combination with an immuno-stimulant (such as mineral oil or aluminum precipitate and heat killed bacteria or lipopolysaccharide, complete Freund's adjuvant, or incomplete Freund's adjuvant). The animals immunized are not particularly limited as long as their cells can be used to form a hybridoma, and include mouse, rat, hamster, guinea pig, rabbit, dog, monkey, sheep, goat, chicken, duck, and the like, preferably mouse or rat, and more preferably mouse. For example, 0.1 to 1000 µg of the immunogen can be administered to an animal at once or several times with suitable intervals (typically, once every 1 to 6 weeks immunization is conducted about 2 to 10 times in total) by subcutaneous, intraperitoneal, intravenous, intradermal, intramuscular, or footpad injection. One to two weeks after the last immunization, blood is collected from orbital cavity or tail vein of the immunized animal, and serum from the blood is used to measure antibody titer. The antibodies of the present invention can be obtained by purifying serum from an animal having an adequate antibody titer.

Monoclonal antibodies can be obtained from culture of a hybridoma obtained by fusing a myeloma cell with an antibody-producing cell derived from an immunosensitized animal immunized as described above. The fusion method can include, for example, the method described by Milstein, et al. (Galfre, G. & Milstein, C. (1981) Methods Enzymol., 73: 3-46). Antibody-producing cells can be taken from spleen, pancreas, lymph node, or peripheral blood derived from mouse or rat that is immunized according to the method as described above and has an adequate antibody titer. Myeloma cells to be used are cells derived from mammals such as, for example, mouse, rat, guinea pig, hamster, rabbit, or human, and are not particularly limited as long as the cells can proliferate in vitro. The cells can include, for example, P3-X63Ag8 (X63) (Nature, 256, 495, 1975), P3/NS1/1-Ag4-1 (NS1) (Eur. J. Immunol., 6, 292, 1976), P3X63Ag8U1 (P3U1) (Curr. Top. Microbiol. Immunol., 81, 1, 1978), P3X63Ag8.653 (653) (J. Immunol., 123, 1548, 1979), Sp2/0-Ag14 (Sp2/O) (Nature, 276, 269, 1978), Sp2/O/FO-2 (FO-2) (J. Immunol. Methods, 35, 1, 1980), and the like. The cells are preferably cells from an animal species that is the same as the animal species from which the antibody-producing cells are derived, and are more preferably cells from an animal strain that is the same as the animal strain from which the antibody-producing cells are derived. For example, myeloma cells from mouse are preferably P3U1 or P3X63-Ag8-653.

Methods of preparing a hybridoma by fusing an antibody-producing cell with a myeloma cell include a method using polyethylene glycol (hereinafter referred to as "PEG") (PEG method), a method using Sendai virus, a method using an electrofusion apparatus, and the like. Hybridoma cells producing monoclonal antibodies can be sorted by a known method or a method similar to the known method. Generally, hybridoma cells can be sorted by selectively grown in a medium for animal cells supplemented with HAT (hypoxanthine-aminopterin-thymidine), typically for 5 days to 3 weeks, preferably for 1 to 2 weeks.

After the culture, the culture supernatant is collected, and clone producing an antibody highly specifically binding to the toxic conformer of Aβ is selected by ELISA or the like. The selected clone is subjected to limiting dilution repeatedly for 1 to 5 times to obtain a single cell. The antibody produced by the single clonal cell can be screened for highly specific binding to the toxic conformer of Aβ to select cells producing antibodies stably exhibiting the high specificity. Antibodies highly specifically binding to the toxic conformer of Aβ of the present invention can be screened by repeatedly selecting clonal cells for high binding capacity to Aβ mutant having β-turn structure at positions 22 and 23 (E22P-Aβ42) (K. Murakami, et al. (2003) J. Biol. Chem., 278: 46179-46187) and low binding capacity to other Aβ structures (e.g., one or more or all peptides selected from A21P-Aβ42, D23P-Aβ42, V24P-Aβ42, G25P-Aβ42, M35P-Aβ42, and E22V-Aβ42), wherein high specificity is prioritized over high avidity.

The antibodies thus obtained can be purified to homogeneity. The antibodies can be separated and purified by methods generally used to separate and purify proteins. The antibodies can be separated and purified by using a technique selected from, for example, column chromatography such as affinity chromatography, filter, ultrafiltration, salt precipitation, dialysis, SDS polyacrylamide gel electrophoresis, isoelectric focusing electrophoresis, and the like, or by using a combination thereof appropriately (Antibodies: A Laboratory Manual. Ed Harlow and David Lane, Cold Spring Harbor Laboratory, 1988). Columns used for affinity chromatography can include such as protein A column and protein G column. Furthermore, regardless of antibody class, E22P-Aβ42-immobilized column, ion exchange chromatography, hydrophobic interaction chromatography, and the like can be also used.

An antibody or immunoreactive fragment thereof of the present invention can be obtained by designing the amino acid sequence of: an antibody or immunoreactive fragment thereof that comprises VH having an amino acid sequence encoded by a nucleic acid sequence which can hybridize with a nucleic acid sequence encoding the amino acid sequence of SEQ ID NO: 4 under a stringent condition, and comprises VL having an amino acid sequence encoded by a nucleic acid sequence which can hybridize with a nucleic acid sequence encoding the amino acid sequence of SEQ ID NO: 11 under a stringent condition; an antibody or immunoreactive fragment thereof that comprises VH having an amino acid sequence having 80%, 85%, 90%, 95%, 98%, or 99% identity to the amino acid sequence of SEQ ID NO: 4, and VL having an amino acid sequence having 80%, 85%, 90%, 95%, 98%, or 99% identity to the amino acid sequence of SEQ ID NO: 11; an antibody or immunoreactive fragment thereof that comprises CDRH1, CDRH2, and CDRH3 having the amino acid sequences of SEQ ID NOs: 5, 6, and 7, respectively, and CDRL1, CDRL2, and CDRL3 having the amino acid sequences of SEQ ID NOs: 12, 13, and 14, respectively, and by preparing DNA fragments encoding thus designed amino acid sequences, followed by inserting the prepared DNA fragments into an expression vector, and transfecting the vector into suitable host cells to express the antibody or fragment, which is then subjected to screening for highly specific binding to the toxic conformer of Aβ according to the method as described above.

The antibodies of the present invention can be also produced, for example, by synthesizing DNA encoding the amino acid sequence set forth in any of SEQ ID NO: 2 (heavy chain with signal peptide), SEQ ID NO: 3 (heavy chain without signal peptide), and SEQ ID NO: 4 (heavy chain variable region) and DNA encoding the amino acid sequence set forth in any of SEQ ID NO: 9 (light chain with signal peptide), SEQ ID NO: 10 (light chain without signal peptide), and SEQ ID NO: 11 (light chain variable region), or synthesizing DNA having the nucleic acid sequences of SEQ ID NOs: 1 and 8, and obtaining H chain and L chain having the amino acid sequence of SEQ ID NO: 2 to SEQ ID NO: 4 and SEQ ID NO: 9 to 11, respectively, or variable regions of the H chain and L chain, respectively from the DNAs.

### (Production of humanized chimeric antibody)

When an antibody of the present invention is a humanized chimeric antibody, the antibody can be obtained by preparing DNA encoding VH and VL of a non-human animal monoclonal antibody highly specifically recognizing the toxic conformer of Aβ (e.g., IBL-102 monoclonal antibody), linking the DNA to cDNA encoding the constant region of human immunoglobulin, inserting the linked DNA into an expression vector, transfecting the vector into suitable host cells, and allowing the DNA to be expressed (Morrison, S. L. et al., Proc. Natl. Acad. Sci. USA, 81, 6851-6855, 1984.

### (Production of humanized antibody)

When an antibody of the present invention is a humanized antibody, the antibody can be obtained by constructing the DNA encoding V region in which the amino acid sequences encoding CDRs of VH and VL of a non-human animal monoclonal antibody specifically recognizing the toxic conformer of Aβ (e.g., IBL-102 monoclonal antibody) are grafted onto FRs of VH and VL of human antibody, and linking the constructed DNA to cDNA encoding the constant region of human immunoglobulin, inserting the linked DNA into an expression vector, transfecting the vector into suitable host cells, and allowing the DNA to be expressed (see L. Rieohmann, et al., Nature, 332, 323, 1988: Kettleborough, C. A. et al., Protein Eng., 4, 773-783, 1991; Clark M., Immunol. Today., 21, 397-402, 2000). The CDRs of the non-human animal monoclonal antibody can be determined by comparing the amino acid sequence predicted from the DNA sequence encoding VH and VL of the non-human animal monoclonal antibody obtained by using the method as described above with total amino acid sequences of VH and VL of known antibodies. Amino acid sequences of known antibodies can be obtained from registration in a database such as Protein Data Bank. The FR of the humanized antibody is not particularly limited as long as the grafted antibody has the effect of the present invention. The FRs of the humanized antibody are preferably FRs of a human antibody that gives a conformation to the variable region (hereinafter referred to as "V region") of the humanized antibody that is similar to the conformation of the V region of the CDR origin non-human animal monoclonal antibody, or preferably FRs of a human antibody that are very homologous to the amino acid sequence of the FRs of the non-human animal monoclonal antibody. In a humanized antibody, some amino acids consisting FRs originated from human antibody (especially amino acids sterically adjacent to CDRs) may be optionally substituted with FR sequence of the CDR origin non-human animal monoclonal antibody (see Queen, et al., United States Patent No. 5585089). The DNA sequence encoding V region of the humanized antibody can be designed as the DNA sequence encoding the amino acid sequence wherein the amino acid sequence of the CDRs of the non-human animal monoclonal antibody are linked to the amino acid sequence of the FRs of human antibody. DNA encoding V region of a humanized antibody can be produced from the designed DNA sequence using a method well known to those skilled in the art.

### (Human Antibodies)

Human antibodies can be obtained, for example, by using human antibody phage libraries or transgenic mice producing human antibodies (Tomizuka, et al., Nature Genet., 15, 146-156 (1997)). For human antibody phage libraries, the desired clones can be obtained, for example, by immobilizing E22P-Aβ42 on a solid phase, contacting a phage antibody library to the solid phase, washing and removing unbound phages, and then collecting the bound phages (panning). The transgenic mice producing human antibodies are mice that have knocked-out endogenous immunoglobulin (Ig) genes and have transferred Ig genes of human antibody. Human antibodies specifically recognizing the toxic conformer of Aβ can be obtained by immunizing the transgenic mice producing human antibodies with an antigen (preferably, E22P-Aβ42) according to the method for producing the antibody of the present invention as described above.

### (Cross-Reaction Rate)

For example, the cross-reaction rate of the antibody of the present invention between the main target (E22P-Aβ42) and any number of one or more (including all) peptides selected from A21P-Aβ42, E22V-Aβ42, D23P-Aβ42, V24P-Aβ42, G25P-Aβ42, and M35P-Aβ42 may be 5% or less, 3% or less, or 1% or less. Alternatively, the respective cross-reaction rates of the antibody of the present invention between the main target (E22P-Aβ42) and any number of one or more (including all) peptides selected from A21P-Aβ42, E22V-Aβ42, D23P-Aβ42, V24P-Aβ42, G25P-Aβ42, and M35P-Aβ42 may be 0.3% or less, 0.3% or less, 0.5% or less, 0.4% or less, 0.6% or less, and 0.7% or less, respectively, and further may be 0.27% or less, 0.28% or less, 0.50% or less, 0.33% or less, 0.53% or less, and 0.68% or less, respectively.

Accordingly, for example, the selection of an antibody can be performed according to the cross reactivity between the toxic conformer of Aβ and Aβs having other structures (e.g., A21P-Aβ42 and E22V-Aβ42). In other words, an antibody having the cross-reaction rate included in the range as described above can be selected as the antibody of the present invention. The cross-reaction rate can be determined in a desired measurement system by generating a standard curve for a test antibody using the toxic conformer of Aβ (e.g., E22P-Aβ42 or E22P-Aβ40) as a standard; obtaining a measured value (e.g., absorbance) for the test antibody using a non-toxic conformer (e.g., A21P-Aβ42 and E22V-Aβ42); comparing the measured value with the standard curve generated using the toxic conformer of Aβ to determine the calculated concentration of the non-toxic conformer; and calculating the following formula: (calculated concentration (the observed value)) / (the actual concentration of the non-toxic conformer added) x 100 (%). The measurement method used for determination of the cross-reaction rate is not particularly limited as long as it is a known measurement method using an antibody molecule, and can be performed according to the method for measuring the toxic conformer of Aβ as described below.

### (Nucleic Acids, Vectors, Host Cells)

Nucleic acids of the present invention can be obtained by cloning a nucleic acid from the antibody-producing hybridoma obtained as described above, or appropriately designing a nucleic acid sequence based on the amino acid sequence of the antibody or immunoreactive fragment thereof obtained as described above. Vectors of the present invention can be obtained by appropriately inserting the obtained nucleic acid into a vector suitable for expression. The vectors of the present invention may comprise a region required for expression (such as a promoter, enhancer, and terminator) in addition to nucleic acids of the present invention. Host cells of the present invention can be obtained by transfecting the vector of the present invention into proper cell lines (e.g., animal cells, insect cells, plant cells, yeasts, and microorganisms such as Escherichia coli).

### (Labels)

A label can be conjugated with the antibodies or immunoreactive fragments thereof using a common method in the art. For example, when fluorescently labeled, a protein or peptide can be conjugated with a label by washing the protein or peptide with a phosphate buffer; adding a dye prepared in DMSO, a buffer, or the like; mixing together; and then incubating for 10 minutes at room temperature. Commercially available labeling kit, including a biotin labeling kit (Biotin Labeling Kit-NH2, Biotin Labeling Kit-SH: DOJINDO LABORATORIES), an alkaline phosphatase labeling kit (Alkaline Phosphatase Labeling Kit-NH2, Alkaline Phosphatase Labeling Kit-SH: DOJINDO LABORATORIES), a peroxidase labeling kit (Peroxidase Labeling Kit-NH2, Peroxidase Labeling Kit-NH2: DOJINDO LABORATORIES), a phycobiliprotein labeling kit (Allophycocyanin Labeling Kit-NH2, Allophycocyanin Labeling Kit-SH, B-Phycoerythrin Labeling Kit-NH2, B-Phycoerythrin Labeling Kit-SH, R-Phycoerythrin Labeling Kit-NH2, R-Phycoerythrin Labeling Kit-SH: DOJINDO LABORATORIES), a fluorescently labeling kit (Fluorescein Labeling Kit-NH2, HiLyte Fluor (R) 555 Labeling Kit-NH2, HiLyte Fluor (R) 647 Labeling Kit-NH2: DOJINDO LABORATORIES), DyLight547, DyLight647 (Techno Chemical Corporation), Zenon (R) Alexa Fluor (R) antibody labeling kit, Qdot (R) antibody labeling kit (Invitrogen), and EZ-Label Protein Labeling Kit (Funakoshi Co., Ltd.), can be also used to label antibodies. Labeled antibodies or fragments thereof can be detected by appropriately using instruments suitable for the labels.

### (Kits)

The present invention also relates to a kit for measuring the toxic conformer of Aβ, comprising an antibody or immunoreactive fragment thereof of the present invention as described above. The kit of the present invention can be manufactured depending on a purpose by using any technique commonly used by those skilled in the art with using an antibody or immunoreactive fragment thereof produced according to the method as described above.

### (Method for detecting and/or measuring the toxic conformer of Aβ)

In another aspect, the present invention relates to methods for detecting and/or measuring the toxic conformer of Aβ. The methods for detecting and/or measuring the toxic conformer of Aβ of the present invention basically comprises contacting a sample with an antibody or immunoreactive fragment thereof of the present invention, and detecting and/or measuring the toxic conformer of Aβ bound to the antibody or immunoreactive fragment thereof. The methods for detecting and/or measuring of the present invention may be performed either in vitro or in vivo, preferably in vitro. For example, the methods for detecting and/or measuring of the present invention can be performed according to any known detection and/or measurement method using an antibody molecule.

More specifically, the method for detection of the present invention includes, for example, a method for determining the presence or absence of the toxic conformer of amyloid β in a sample, comprising:
(a) contacting the sample with an antibody or immunoreactive fragment thereof that highly specifically recognizes a turn structure at amino acid positions 22 and 23 of Aβ but does not recognize any turn structure at other sites of Aβ,
(b) detecting the toxic conformer of amyloid β bound to the antibody or immunoreactive fragment thereof; and
(c) determining that the toxic conformer of amyloid β is present in the sample when the toxic conformer of amyloid β is detected in the previous step, or determining that the toxic conformer of amyloid β is absent in the sample when the toxic conformer of amyloid β is not detected in the previous step.

The method for measurement of the present invention also include, for example, a method for determining a level of the toxic conformer of amyloid β in a sample, comprising:
(a) contacting the sample with an antibody or immunoreactive fragment thereof that highly specifically recognizes a turn structure at amino acid positions 22 and 23 of Aβ but does not recognize any turn structure at other sites of Aβ;
(b) measuring the amount of the toxic conformer of amyloid β bound to the antibody or immunoreactive fragment thereof; and
(c) calculating the level of the toxic conformer of amyloid β in the sample from the amount of the toxic conformer detected.

When the method for measurement of the present invention is performed quantitatively, the concentration of the toxic conformer of amyloid β can be determined by generating a standard curve using a standard (e.g., E22P-Aβ42) serially diluted to appropriate known concentrations which is measured at the same time with or separately from the measurement of the sample, and calculating the concentration of the toxic conformer of amyloid β from the measured value for the sample based on the standard curve.

In this specification, the steps of "contacting the sample with an antibody or immunoreactive fragment thereof" and "detecting or quantifying the toxic conformer of amyloid β in the sample" can be performed, for example, by a sandwich ELISA. Specifically, the steps can be performed by contacting a test sample (sample) with an antibody or immunoreactive fragment thereof of the present invention immobilized onto a solid phase, washing, adding a labeled antibody that can bind to an target substance (the toxic conformer of amyloid β), washing to remove unbound antibodies, and detecting the label of the antibody or measuring the amount (or intensity) of the label. An immunochromatography can be performed by contacting the sample with a labeled antibody that can bind to a non-immobilized target substance (the toxic conformer of amyloid β), then contacting the mixture with a carrier immobilized with the antibody or immunoreactive fragment thereof of the present invention at a specific site, and detecting the labeled antibody bound at the site or measuring the amount (or intensity) of the label at the site. In this paragraph, a labeled antibody or immunoreactive fragment thereof of the present invention may be used instead of the labeled antibody that can bind to a target substance (the toxic conformer of amyloid β), and an antibody that can bind to a target substance (the toxic conformer of amyloid β) may be used instead of the antibody or immunoreactive fragment thereof of the present invention.

Alternatively, the antibodies or immunoreactive fragments thereof of the present invention can be used to detect and/or measure the toxic conformer of Aβ in vivo. For example, the present invention includes a method for detecting or measuring the toxic conformer of Aβ in a living body, comprising administering an antibody or immunoreactive fragment thereof of the present invention labeled with a fluorescent or radioactive material to a living body, and detecting and/or measuring the label in the living body. For example, in an applied manner, the present invention may be a method for determining the existing location of the toxic conformer of Aβ in a living body, comprising administering the antibody or immunoreactive fragment thereof of the present invention which is labeled with a fluorescent or radioactive material to a living body, and detecting the label in the living body to determine the location of the labeled antibody. The present invention may be a method for determining the amount of the toxic conformer of Aβ in a living body, comprising administering an antibody or immunoreactive fragment thereof of the present invention which is labeled with a fluorescent or radioactive material to a living body, measuring the amount or intensity of the label in the living body, and determining the level of the toxic conformer of Aβ from the measured amount or intensity of the label. The present invention may be a method for determining the location and the amount of the toxic conformer of Aβ in a living body, comprising administering the antibody or immunoreactive fragment thereof of the present invention which is labeled with a fluorescent or radioactive material to the living body, measuring the location and the amount or intensity of the label in the living body, determining the level of the toxic conformer of Aβ from the measured amount or intensity of the label, and correlating the location with the level of the toxic conformer of Aβ.

### (Method for diagnosing AD)

Toxic conformers of Aβ are associated with development and exacerbation of AD. It has been believed so far that the level of Aβ40 or Aβ42 (the total amount of Aβ) is associated with development and exacerbation of AD, but it has been suggested that increase in Aβ with a specific conformation (toxic conformer) rather than the total amount of Aβ is associated with development of AD. Aβ is known to be able to have various conformations, and the antibody and immunoreactive fragment thereof of the present invention highly specifically recognize only the toxic conformer of Aβ. Accordingly, the antibody and immunoreactive fragment thereof of the present invention can achieve more precise diagnosis of AD. Thus, the present invention includes an agent for diagnosing AD comprising the above described antibody and immunoreactive fragment thereof of the present invention as an active ingredient. The present invention also includes a method for diagnosing AD, a method for providing information for diagnosing AD, a method for monitoring the condition or progression of AD, and a method for determining therapeutic efficacy of candidate therapeutic agents for AD (hereinafter collectively referred to as "diagnostic method and the like of the present invention"), comprising measuring the toxic conformer of Aβ using the above described antibody or immunoreactive fragment thereof of the present invention. Throughout this specification, the term "diagnosis of AD" and "diagnosing AD" may be read as "providing information for diagnosing AD", "monitor of the condition or progression of AD" and "monitoring the condition or progression of AD", or "determination of therapeutic efficacy of candidate therapeutic agents for AD" and "determining therapeutic efficacy of candidate therapeutic agents for AD" except for cases in which such interpretation is inconsistent. In another aspect, the present invention includes a use of the antibody or immunoreactive fragment thereof of the present invention for diagnosing AD (or monitoring the condition or progression of AD, or determining therapeutic efficacy of candidate therapeutic agents for AD). The present invention also relates to a use of the antibody or immunoreactive fragment thereof of the present invention for manufacturing a medicament or a composition for diagnosing AD (or monitoring the condition or progression of AD, or determining therapeutic efficacy of candidate therapeutic agents for AD).

As used herein, the term "diagnosis" may be interpreted not only as determination of whether or not the patient has AD and/or determination of the severity of AD (diagnosis in a narrow sense), but also as provision of information for diagnosing AD or monitoring of the condition or progression of AD depending on the intended purpose, except for cases in which such interpretation is particularly inconsistent. In the diagnostic method of the present invention, the detection of the presence or absence or the measurement of the level of the toxic conformer of Aβ can be performed, for example, according to the method for measuring the toxic conformer of Aβ as described above.

The diagnostic method and the like of AD of the present invention comprise measuring the toxic conformer of Aβ in a sample, and diagnosing AD using the measured amount of the toxic conformer of Aβ as an indicator. More specifically, the diagnostic method of the present invention comprise a method for diagnosing AD, comprising the steps of:
a) contacting a sample from a subject with at least one antibody or immunoreactive fragment thereof that highly specifically recognizes turn structure at amino acid positions 22 and 23 of Aβ but does not recognize any turn structure at other sites of Aβ,
b) measuring the toxic conformer of Aβ bound to the antibody or immunoreactive fragment thereof, and determining the level of the toxic conformer of Aβ from the measured value, and
c) evaluating the presence or absence of AD, or stage of AD development in the subject from the determined level of the toxic conformer of Aβ,
wherein a subject evaluated to have a higher level of the toxic conformer of Aβ is determined as having AD or as having a higher severity of AD.

When the diagnostic method and the like of the present invention is a method for providing information for diagnosing AD, it includes a method for providing information for diagnosing AD, comprising the steps of:
a) contacting a sample from a subject with at least one antibody or immunoreactive fragment thereof that highly specifically recognizes turn structure at amino acid positions 22 and 23 of Aβ but does not recognize any turn structure at other sites of Aβ,
b) measuring the toxic conformer of Aβ bound to the antibody or immunoreactive fragment thereof and determining the level of the toxic conformer of Aβ from the measured value, and
c) providing information of a result of evaluation for the presence or absence of AD, or stage of AD development in the subject evaluated from the determined level of the toxic conformer of Aβ,
wherein a subject evaluated to have a higher level of the toxic conformer of Aβ is determined as having AD or as having a higher severity of AD.

When the diagnostic method and the like of the present invention is a method for monitoring the condition or progression of AD, it includes a method for monitoring the condition or progression of AD, comprising:
a) contacting a sample from a subject with at least one antibody or immunoreactive fragment thereof that highly specifically recognizes turn structure at amino acid positions 22 and 23 of Aβ but does not recognize any turn structure at other sites of Aβ,
b) measuring the binding of the toxic conformer of Aβ in the sample to the antibody or immunoreactive fragment thereof, and determining the level of the toxic conformer of Aβ from the measured value, and
c) comparing the determined level of the toxic conformer of Aβ with that previously and separately determined using the same method, and evaluating the alteration of AD development in the subject,
wherein AD in the subject is evaluated to be progressed when the determined level of the toxic conformer of Aβ is higher than that previously and separately determined using the same method.

When an antibody or immunologically binding fragment thereof that specifically binds to the toxic conformer of Aβ is administered to a living body in the diagnostic methods of the present invention, the diagnosis can be performed by diagnostic imaging. In employing diagnostic imaging, the diagnostic method and the like of the present invention includes a method for diagnosing AD, comprising the steps of:
a) producing an image of a portion or larger part of an antibody or immunoreactive fragment thereof that highly specifically recognizes turn structure at amino acid positions 22 and 23 of Aβ but does not recognize any turn structure at other sites of Aβ in a subject that received at least one antibody or immunoreactive fragment thereof by a diagnostic imaging device (e.g., scintigraphy, PET, or SPECT modality), and detecting and/or measuring the antibody or immunoreactive fragment thereof in the living body,
b) determining the presence or absence, the localization, and/or the level of the toxic conformer of Aβ in the subject's body from the location and/or value (e.g., intensity) of the detected and/or measured antibody or immunoreactive fragment thereof, and
c) evaluating a subject determined the presence of the toxic conformer of Aβ or determined to have a higher level of the toxic conformer of Aβ, a subject whose toxic conformer of Aβ was determined in the brain, or a subject determined to have a high level of the toxic conformer of Aβ in the brain, as having AD or as having a higher severity of AD.

Alternatively, the diagnostic methods for AD of the present invention comprise measuring the toxic conformer of Aβ in a sample and utilize the ratio of the amount of the toxic conformer of Aβ to the total amount of Aβ as an indicator for diagnosis. More specifically, the diagnostic method and the like of the present invention include a method for diagnosing AD, comprising the steps of:
a) contacting a sample from a subject with at least one antibody or immunoreactive fragment thereof of the present invention,
b) measuring a level of an antigen that can bind to the antibody or immunoreactive fragment thereof of the present invention, bound to the antibody or immunoreactive fragment thereof of the present invention,
c) contacting the sample from the subject with an anti-Aβ antibody that is not specific for Aβ structure, and measuring the amount of the bound complex to determine the total amount of Aβ in the sample from the subject, and
d) calculating the ratio of the level of an antigen bound to the antibody or immunoreactive fragment thereof of the present invention to the total level of Aβ in the sample from the subject to evaluate the presence or absence of AD, or stage of AD in the subject from the calculated ratio,
wherein the subject is evaluated as highly likely to have AD when the ratio of the level of an antigen bound to the antibody or immunoreactive fragment thereof of the present invention to the total level of Aβ is higher than the ratio in a sample from a healthy subject.

The method for diagnosis using the ratio of the amount of the toxic conformer of Aβ to the total amount of Aβ as an indicator can be conducted as a method for providing information for diagnosing AD, and a method for monitoring the condition or progression of AD as described above.

The "evaluating step" in the diagnostic method and the like of the present invention can be performed by using the level of toxic conformer of Aβ or the ratio of the amount of toxic conformer of Aβ to the total amount of Aβ. When the level of toxic conformer of Aβ or the ratio of the amount of toxic conformer of Aβ to the total amount of Aβ is used in the diagnostic method and the like of the present invention, the evaluating step can be performed by comparing the level of toxic conformer of Aβ or the ratio of the amount of toxic conformer of Aβ to the total amount of Aβ in a subject with the level of toxic conformer of Aβ or the ratio of the amount of toxic conformer of Aβ to the total amount of Aβ in a healthy subject, a patient having no AD, and/or a patient that is believed to have no AD (hereinafter collectively referred to as "negative control"). When the level of toxic conformer of Aβ or the ratio of the amount of toxic conformer of Aβ to the total amount of Aβ in a sample from a subject is higher than the level of toxic conformer of Aβ or the ratio of the amount of toxic conformer of Aβ to the total amount of Aβ in a sample from such a negative control, "the level of toxic conformer of Aβ or the ratio of the amount of toxic conformer of Aβ to the total amount of Aβ is high", that is, the subject can be evaluated to have AD or to have a higher severity of AD. When the level of toxic conformer of Aβ or the ratio of the amount of toxic conformer of A|3 to the total amount of Aβ in a sample from a subject is not higher than (i.e., is equal to or lower than) the level of toxic conformer of Aβ or the ratio of the amount of toxic conformer of Aβ to the total amount of Aβ in a sample from such a negative control, "the level of toxic conformer of Aβ or the ratio of the amount of the toxic conformer of Aβ to the total amount of Aβ is not high", that is, the subject can be evaluated to have no AD or to have a low severity of AD.

Throughout this specification, a statistical analysis can be used to determine whether the level of toxic conformer of Aβ or the ratio of the amount of toxic conformer of Aβ to the total amount of Aβ in a sample is higher than the level of toxic conformer of Aβ or the ratio of the amount of toxic conformer of Aβ to the total amount of Aβ in a control sample, respectively. Statistical significance is determined by comparing two or more samples and calculating confidence interval and/or p-value (Dowdy and Wearden, Statistics for Research, John Wiely & Sons, NewYord, 1983). The confidence interval in the present invention may be, for example, 90%, 95%, 98%, 99%, 99.5%, 99.9%, or 99.99%. The p-value in the present invention may be, for example, 0.1, 0.05, 0.025, 0.02, 0.01, 0.005, 0.001, 0.0005, 0.0002, or 0.0001.

Throughout this specification, the level of toxic conformer of Aβ in a "control" (including positive and negative control) can be measured in a way similar to the measurement of the level of toxic conformer of Aβ in a subject. Alternatively, if information about the level of toxic conformer of Aβ previously measured in the control is given, the information can be used as the level of toxic conformer of Aβ or the ratio of the amount of the toxic conformer of Aβ to the total amount of Aβ in the control.

The evaluating step may be also performed, for example, by defining a threshold level or a threshold ratio for the level of toxic conformer of Aβ or the ratio of the amount of toxic conformer of Aβ to the total amount of Aβ as an indicator of the presence or absence of the affected AD or the severity of the affected AD based on the level of toxic conformer of Aβ or the ratio of the amount of toxic conformer of Aβ to the total amount of Aβ that is previously obtained in a negative control and the level of toxic conformer of Aβ or the ratio of the amount of toxic conformer of Aβ to the total amount of Aβ (and information about the severity of the developed AD) in an AD patient or a patient that is believed to have AD (hereinafter collectively referred to as "positive control") and comparing the defined threshold level or ratio with the level of toxic conformer of Aβ in a sample from a subject.

Particularly, when "the severity of the affected AD" is used for diagnosis, the determining step can be performed simply by using the control severity of AD corresponding to a level of toxic conformer of Aβ or a ratio of the amount of toxic conformer of Aβ to the total amount of Aβ similar to the levels of toxic conformer of Aβ or the ratios of the amount of toxic conformer of Aβ to the total amount of Aβ that are previously obtained in negative control and positive control. Alternatively, the step of determining "the severity of the affected AD" can be performed by establishing some groups (classes) (e.g., severe, moderate, mild, undeveloped, and the like, or the degree of cognitive impairment) having similar severity of the affected AD. In other words, the severity of the affected AD is assigned to each class (group) by defining two or more threshold levels of toxic conformer of Aβ based on the level of toxic conformer of Aβ or the ratio of the amount of the toxic conformer of Aβ to the total amount of Aβ previously obtained in negative control and positive control and information about AD severity in positive control; establishing at least 3 groups, the group having the upper limit equal to the lowest threshold level or ratio of the amount of toxic conformer of Aβ to the total amount of Aβ, the group having the upper limit and the lower limit equal to the two nearest neighboring threshold levels or ratios of the amount of toxic conformer of Aβ to the total amount of Aβ, and the group having the lower limit equal to the highest threshold level; and considering the severity class of each group as the severity (such as healthy, undeveloped, mild AD, moderate AD, or severe AD) of the control belonging to the each group. The severity of AD in a subject may be determined by measuring the level of toxic conformer of Aβ in a sample from the subject, determining the class corresponding to the level, and determining the AD severity class corresponding to the determined class. The "information about the severity of the developed AD" in the description above can be obtained using previously established various indicators regarding the developed AD. In the classification, when a group has a higher level of toxic conformer of Aβ or a higher ratio of the amount of toxic conformer of Aβ to the total amount of Aβ, the group is classified into a higher severity class of the developed AD.

The threshold level as described above can be defined to provide the sensitivity of 50, 55, 60, 65, 70, 75, 80, 85, 90, 91, 92, 93, 94, 95, 96, 97, or 98% or more. The threshold level can be also defined to provide the specificity of 40, 45, 50, 55, 60, 65, 70, 75, 80, 85, 90, 91, 92, 93, 94, 95, 96, 97, or 98% or more.

When the diagnostic methods of the present invention are a method for determining therapeutic efficacy of a candidate therapeutic agent for AD, the diagnostic methods include a method for determining therapeutic efficacy of a candidate therapeutic agent for AD, comprising:
a) contacting an antibody or immunoreactive fragment thereof that highly specifically recognizes at least one turn structure at amino acid positions 22 and 23 of Aβ but does not recognize any turn structure at other sites of Aβ with a sample from a subject that has not received a candidate therapeutic agent for AD and/or a sample from the subject that has received a candidate therapeutic agent for AD,
b) measuring the toxic conformer of Aβ bound to the antibody or immunoreactive fragment thereof to determine the level of toxic conformer of Aβ or the ratio of the amount of toxic conformer of Aβ to the total amount of Aβ from the measurement, and
c) comparing the levels of toxic conformer of Aβ or the ratios of the amount of toxic conformer of Aβ to the total amount of Aβ determined in the sample from the subject that has not received a candidate therapeutic agent for AD and the sample from the subject that has received a candidate therapeutic agent for AD to determine efficacy of the candidate therapeutic agent for AD in the subjects,
wherein the candidate therapeutic agent for AD is determined as having therapeutic efficacy for AD when the level of toxic conformer of Aβ or the ratio of the amount of toxic conformer of Aβ to the total amount of Aβ in the sample from the subject that has received the candidate therapeutic agent for AD is lower than that in the sample from the subject that has not received the candidate therapeutic agent for AD.

The diagnostic methods of the present invention may be performed in combination with other indicators that have been previously used for AD.

As used herein, "AD" means an AD or AD-type dementia diagnosed according to the diagnostic criteria of Alzheimer-type dementia based on the DSM-IV classification (American Psychiatric Association: Diagnostid and statistical manual of mental disorders, 4th ed (DSM-IV). American Psychiatric Association, Washington D.C., 1994) or the clinical diagnostic criteria of Alzheimer's disease of the NINCDS-ADRDA Work Group (McKahann G, et al.: Clinical diagnosis of Alzheimer's disease-report of the NINCDS-ADRDA Work Group under the auspices of Department of Health and Human Services Task Force on Alzheimer's disease. Neurology 34:939, 1984). Specifically, a patient may be diagnosed as AD or AD-type dementia when the patient exhibits memory impairment and cognitive dysfunction (aphasia, apraxia, agnosia, or executive functions); markedly impaired social or occupational functions due to cognitive impairment; and the progress characterized by a slow development of the disease and persistent cognitive decline; and has no dementia due to other central nervous system diseases, systemic diseases, or exogenous substances; the impairments do not occur only for the duration of impaired consciousness (delirium); and are unable to be explained by other primary psychiatric diseases. Alternatively, a patient may be diagnosed as AD or AD-type dementia when the patient has two or more cognitive impairments, has memorization and other cognitive functions progressively exacerbated, has no impaired consciousness, and has no systemic diseases or brain diseases other than AD caused by dementia. Alternatively, AD may be histopathologically estimated using brain obtained in biopsy or autopsy.

### (Levels)

Throughout this specification, a "level" means an indicator of abundance converted into a numerical form and includes, for example, concentration, amount, or any other indicator that can be used instead of concentration and amount. Thus, a level may be a measurement value itself such as fluorescence intensity or a value converted into concentration. A level may be also an absolute numerical value (e.g., abundance or abundance per unit area) or a relative numerical value to an optionally defined control.

### (Samples)

Samples used in all the methods of the present invention as described above are not particularly limited as long as the methods of the present invention can measure toxic conformers of Aβ in the samples, and the samples can be appropriately selected depending on the purpose of use. For example, as used herein, "samples" include a cell culture supernatant, a cell lysate, a tissue specimen or fluid specimen taken from a subject as a biopsy, and the like. Bodily fluids from subjects can include, for example, bodily fluids such as blood, plasma, serum, lymphatic fluid, urea, serosity, spinal fluid, cerebral spinal fluid, synovial fluid, aqueous humor, tear, and saliva, or fractions or processing products thereof. Tissue specimen s from subjects can include a brain tissue or a brain tissue lysate. Samples used in the methods of the present invention may be samples from subjects previously processed prior to measurement tests or may be samples themselves taken from subjects. The subjects include human and nonhuman animals. Analyses in the methods of the present invention can be performed qualitatively, quantitatively, or semiquantitatively.

### (Known measurement methods using antibody molecules)

When EIA is adopted for known measurement methods using antibody molecules, binding of an antibody or immunologically binding fragment thereof specifically binding to the toxic conformer of Aβ to the toxic conformer of Aβ in a sample can be measured by contacting the antibody or immunologically binding fragment thereof specifically binding to the toxic conformer of Aβ with the toxic conformer of Aβ in the sample; then allowing the antibody or immunologically binding fragment thereof binding to the toxic conformer of Aβ to bind to a labeled antibody recognizing the antibody or immunologically binding fragment thereof; removing unbound antibodies; and then measuring the formed complex using a method suitable for the labeling agent. When the measurement is performed quantitatively, standardized solutions obtained by serially diluting a standard (E22P-Aβ42) having a known concentration are used to generate a standard curve, and concentrations corresponding to measurements can be calculated from the standard curve.

When the measurement is performed by immunochromatography, toxic conformers of Aβ in a sample can be detected by contacting the sample with a labeled antibody and then performing chromatography via capillary action onto a nitrocellulose membrane to allow the sample and the labeled antibody to bind to the immobilized antibody or immunologically binding fragment thereof specifically binding to the toxic conformer of Aβ. Also, a gold colloid can be used as a label in immunochromatography to visually confirm the binding of the immobilized antibody to the complex formed between the sample and the labeled antibody.

### (Pharmaceutical compositions (therapeutic agents and prophylactic agents) and agents or compositions for detection and/or measurement in vivo)

The antibodies or immunoreactive fragments thereof of the present invention can be used as a pharmaceutical composition (including an agent for treating and preventing AD) or an agent or composition for detection and/or measurement in vivo obtained by formulating the antibody or immunoreactive fragment thereof, which is optionally purified, according to any conventional method. The present invention also includes use of the antibodies or immunoreactive fragments thereof of the present invention for manufacturing a pharmaceutical composition (including an agent for treating and preventing AD) or an agent or composition for detection and/or measurement in vivo. Alternatively, the present invention includes use of the antibodies or immunoreactive fragments thereof of the present invention for treatment or prevention of AD or for detection or measurement in vivo.

For example, pharmaceutical compositions (therapeutic agents or prophylactic agents) or agents or compositions for detection or measurement in vivo of the present invention can be used as an injection and include dosage forms such as an intravenous injection, a subcutaneous injection, an intradermal injection, an intramuscular injection, and an intravenous infusion. These injections can be prepared according to any known method, for example, by dissolving, suspending, or emulsifying the antibody or the like in a sterile aqueous or oleaginous solution used in a common injection. Aqueous solutions for injection that can be used include, for example, physiological saline and isotonic solutions containing glucose, sucrose, mannitol, and other adjuvants and can be used in combination with suitable dissolution aids, for example, alcohols (e.g., ethanol), polyalcohols (e.g., propylene glycol, polyetheylene glycol), nonionic detergents (e.g., polysorbate 80, polysorbate 20, HCO-50 (polyoxyethylene (50 mol) adduct of hydrogenated castor oil)), and the like. Oleaginous solutions that can be used include, for example, sesame oil, soybean oil, and the like, and can be used in combination with benzyl benzoate, benzyl alcohol, or the like as a dissolution aid. The prepared injection solutions are typically packed in suitable ampules, vials, or syringes. Injection solutions can be also prepared by adding a suitable excipient to the antibody or immunoreactive fragment thereof of the present invention to obtain a lyophilized formulation and dissolving the lyophilized formulation in water for injection, a physiological saline, or the like just before use. It is noted that oral administration may be achieved by devising an antibody fragment or a modified antibody fragment and dosage forms although oral administration of proteins including antibodies is generally difficult due to degradation in the gastrointestinal tract. Formulations for oral administration can include, for example, a capsule, a tablet, a syrup, a granule, and the like.

Pharmaceutical compositions (therapeutic agents or prophylactic agents) or agents or compositions for detection or measurement in vivo of the present invention are suitably prepared in a dosage unit form adapted to the dosage of active ingredient. Such a dosage unit form includes an injection (ampule, vial, or prefilled syringe) and may comprise typically 5 to 500 mg, 5 to 100 mg, and 10 to 250 mg of an antibody or immunoreactive fragment thereof of the present invention per dosage unit form.

Pharmaceutical compositions (therapeutic agents or prophylactic agents) or agents or compositions for detection or measurement in vivo of the present invention may be administered locally or systemically. Routes of administration are not particularly limited, and parenteral or oral administration is performed as described above. Parenteral routes of administration include subcutaneous or intraperitoneal injections or infusions, or injections or infusions into blood vessel (intravenous or intraarterial injection or infusion) or into spinal cord, and preferably administration into blood vessel. Pharmaceutical compositions (therapeutic agents or prophylactic agents) or agents or compositions for detection or measurement in vivo of the present invention may be administered by bolus injection, or continuously or intermittently. For example, administration can be continued for one minute to two weeks.

Dosages of pharmaceutical compositions of the present invention are not particularly limited as long as the dosages result in any desired therapeutic or prophylactic efficacy. The dosages can be appropriately determined depending on the symptom, sex, age, and the like. Dosages of pharmaceutical compositions of the present invention can be determined, for example, by using a therapeutic or prophylactic efficacy for AD as an indicator. For example, when used for prevention and/or treatment in AD patients, the pharmaceutical composition of the present invention is conveniently administered by intravenous injection typically at about 0.01 to 20 mg/kg of body weight, preferably about 0.1 to 10 mg/kg of body weight, and more preferably about 0.1 to 5 mg/kg of body weight for a dose of the active ingredient about 1 to 10 times a month, and preferably about 1 to 5 times a month. In the case of other parenteral administrations and oral administration, the amount calculated based on the above-mentioned amount can be used for administration. When a symptom is particularly severe, the dosage or the number of doses may increase depending on the symptom.

Dosages of agents or compositions for detection or measurement in vivo of the present invention are not particularly limited as long as the dosages allow the desired detection, measurement, or diagnosis. The dosages can be also appropriately determined depending on the symptom, sex, age, and the like. For example, when used for diagnosis in AD patients, the agent or composition for detection or determination in vivo of the present invention can be administered by intravenous injection typically at about 0.01 to 20 mg/kg of body weight, preferably about 0.1 to 10 mg/kg of body weight, and more preferably about 0.1 to 5 mg/kg of body weight for a dose of the active ingredient about one or a few times a month. In the case of other parenteral administrations and oral administration, the amount calculated based on the above-mentioned amount can be used for administration.

The Examples will be shown below to describe the present invention in more detail, but the present invention is not limited to the Examples. It is noted that all references cited throughout the present application are incorporated herein by reference in their entireties.

### [Examples]

### (Example 1) Production of antibody against the toxic conformer having a turn structure at positions 22 and 23 of Aβ42

The maintenance and experiment of mice in the experiment of Example 1 were performed according to the protocols approved by the committee for the protection of animals of Immuno-Biological Laboratories Co., Ltd. The molecular weights of G9C,E22P-Aβ9-35 and E22P-Aβ9-35 were confirmed by MALDI-TOF-MS (K. Murakami, et al. (2010) ACS. Chem. Neurosci., 1: 747-756).

G9C,E22P-Aβ9-35 peptide (CYEVHHQKLVFFAPDVGSNKGAIIGLM: SEQ ID NO: 1) was synthesized according to the reported method (K. Murakami, et al. (2002) Biochem. Biophys. Res. Commun., 294: 5-10; K. Murakami, et al. (2007) ChemBioChem, 8: 2308-2314) and used as an immunogen. The peptide, in which glycine at position 9 is substituted with cysteine, is linked to cattle thyroglobulin, a carrier protein, at the N-terminal potion (Y. Horikoshi, et al. (2004) Biochem. Biophys. Res. Commun., 319: 733-737). BALB/c mice (CHARLES RIVER LABORATORIES JAPAN, INC., Japan) were immunized with 50 mg/mouse of G9C,E22P Aβ9-35 peptide fusion once a week for a month. The resulting clones were incubated at room temperature for one hour in a 96-well Maxisorp plate (Nunc, Denmark) coated with 50 mg/mL of various antibody mutants. The plate was then treated with horseradish peroxidase-conjugated anti-mouse IgG antibody (Sigma, St. Louis, Missouri, United States) and quantified using 3,3',5,5'-tetramethylbenzidine (Pierce, Rockfield, Illinois, United States) or o-phenylenediamine dihydrochloride substrate (Sigma). The resulting 45 clones were screened for high binding capacity to Aβ mutants that tend to have β-turn structure at positions 22 and 23 (E22Q-Aβ42, E22G-Aβ42, E22K-Aβ42, E22P-Aβ42, and D23N-Aβ42) (K. Murakami, et al. (2003) J. Biol. Chem., 278: 46179-46187). The selected clones were subcloned. The less positive or false-positive clones were removed by repeatedly performing the screening. The resulting clones were further screened by Western blot to obtain the clone IBL-102.

### (Example 2) Determination #1 of binding specificity of IBL-102 antibody

The binding capacity of the monoclonal antibody produced by the obtained clone IBL-102 (hereinafter referred to as "IBL-102 antibody") to each of the following antigens was determined by an enzyme immunoassay: 22,23 lactam-Aβ, E22P-Aβ42, E22G-Aβ42, E22G-Aβ40, E22K-Aβ42, E22Q-Aβ40, E22Δ-Aβ42, E22Δ-Aβ40, 25,26 lactam-Aβ, A21P-Aβ42, E22V-Aβ42, D23P-Aβ42, V24P-Aβ42, G25P-Aβ42, M35P-Aβ42, wild-type Aβ38, wild-type Aβ40, wild-type Aβ42, recom.Aβ42, wild-type Aβ43, and wild-type Aβ46. Specifically, 4000, 1000, 250, or 62.5 pg/mL of each of the antigens as described above or 0.1% (w/v) BSA as a control was added to a 96-well plate immobilized (coated) with 82E1 antibody (recognizing residues at positions 1 to 16 of Aβ sequence (not the turn structure portion); see Y. Horikoshi, et al. (2004) Biochem Biophys Res Commun. 2004 Jul 2; 319 (3): 733-7.; the same shall apply hereinafter) in 0.1 M carbonate buffer at 1 µg/well. After standing overnight at 4°C, the reaction solution was removed from wells, and the wells were washed. One hundred µL of anti-Aβ mouse monoclonal antibody IBL-102 labeled with horseradish peroxidase (HRP) was added to each well. After standing for 60 minutes at 4°C, the reaction solution was removed from wells, and the wells were washed. One hundred µL of 3,3',5,5'-tetramethylbenzidine (TMB) substrate solution was added. After standing for 30 minutes at room temperature with protection from light, 100 µL of 1N H₂SO₄ was added to stop the color reaction. Absorbance at 450 nm was measured.

The results obtained by determining binding capacity of IBL-102 antibody to each of the antigens as described above are shown in Table 1. The numerical values in Table 1 represent absorbance at 450 nm. IBL-102 antibody showed highly specific immunoreactivity to E22P-Aβ42 and low affinity to other antigens. This result indicates that IBL-102 antibody very specifically recognizes only Aβ42 having a turn structure at positions 22 and 23. The data of E22P-Aβ42 shown in Table 1 was used to generate a standard curve. From the generated standard curve, the concentration corresponding to the OD value measured using each of A21P-Aβ42, E22V-Aβ42, D23P-Aβ42, V24P-Aβ42, G25P-Aβ42, and M35P-Aβ42 in 4000 pg/mL was determined, wherein the concentration was on the assumption that the antigen used is E22P. The cross-reaction rate, which is the rate (%) of the determined concentration relative to the actually used concentration (4000 pg/mL), was determined. As a result, the cross-reaction rates between the main target (E22P-Aβ42) of IBL-102 antibody and A21P-Aβ42, E22V-Aβ42, D23P-Aβ42, V24P-Aβ42, G25P-Aβ42, and M35P-Aβ42 were 0.27%, 0.28%, 0.50%, 0.33%, 0.53%, and 0.68%, respectively.

**[Table 1]**

| **Antigen** | Amount of antigen (pg/mL) | | | |
|---|---|---|---|---|
| | 4000 | 1000 | 250 | 62.5 |
| 22,23lactam-Aβ42 | 0.057 | 0.011 | -0.001 | -0.005 |
| E22P-Aβ42 | 3.92 | 3.92 | 3.489 | 1.119 |
| E22G-Aβ42 | 0.321 | 0.111 | 0.121 | 0.051 |
| E22G-Aβ40 | 0.55 | 0.074 | 0.009 | -0.007 |
| E22K-Aβ42 | 0.093 | 0.018 | 0.001 | -0.001 |
| E22Q-Aβ40 | 0.863 | 0.092 | 0.01 | -0.007 |
| E22Δ-Aβ42 | 0.25 | 0.118 | 0.163 | 0.031 |
| E22Δ-Aβ40 | 1.818 | 0.437 | 0.194 | 0.116 |
| 25,26lactam-Aβ42 | 0.188 | 0.049 | 0.027 | 0.017 |
| A21P-Aβ42 | 0.193 | 0.047 | 0.011 | -0.003 |
| E22V-Aβ42 | 0.198 | 0.085 | 0.021 | 0.054 |
| D23P-Aβ42 | 0.358 | 0.086 | 0.023 | 0.004 |
| V24P-Aβ42 | 0.233 | 0.116 | 0.05 | 0.064 |
| G25P-Aβ42 | 0.383 | 0.127 | 0.035 | 0.014 |
| M35P-Aβ42 | 0.489 | 0.18 | 0.107 | 0.047 |
| WT-Aβ38 | 0.433 | 0.161 | 0.052 | 0.009 |
| WT-Aβ40 | 0.404 | 0.193 | 0.087 | 0.054 |
| WT-Aβ42 | 0.283 | 0.085 | 0.021 | 0.002 |
| recom.Aβ42 | 0.358 | 0.2 | 0.062 | 0.113 |
| WT-Aβ43 | 0.384 | 0.213 | 0.091 | 0.042 |
| WT-Aβ46 | 0.124 | 0.126 | 0.09 | 0.087 |

### (Example 3) Determination #2 of binding specificity of IBL-102 antibody

The binding capacity of the obtained monoclonal antibody IBL-102 (hereinafter referred to as IBL-102 antibody) to each of the following antigens was determined by an enzyme immunoassay: E22P-Aβ42, E22V-Aβ42, wild-type Aβ42, wild-type Aβ40, and bovine serum albumin (BSA) as a control. Each antigen in a buffer for immobilization (0.1 M carbonate buffer, pH 9.5) was added to a 96-well ELISA plate at concentration of 50 ng/well and allowed to react overnight at 4°C to achieve immobilization. After washing with PBS, the plate was blocked with a blocking solution (1%BSA, 0.05%Tween 20/PBS). After each antigen was immobilized as described above, IBL-102 antibody was diluted to concentrations of 1, 0.5, 0.25, and 0.125 µg/mL and added to each of the immobilized antigens at 50 µL/well. After reacting for 30 minutes at 37°C, the plate was washed, and a labeled antibody (Anti-Mouse IgG (H + L) Goat Fab'-HRP) was added at 50 µL/well. The reaction was continued for 30 minutes at 37°C, and the plate was then washed. A substrate solution for color reaction (o-phenylenediamine dihydrochloride; OPD) was added to trigger the color reaction (for 15 minutes at room temperature with protection from light), and a stop solution (1N H₂SO₄) was added to stop the reaction. Subsequently, absorbance at 450 nm was measured. IBL-101 antibody (#10379, AntiHuman Amyloidβ E22P (11A1) Mouse IgG MoAb, Immuno-Biological Laboratories Co, Ltd., Japan) was similarly tested as a control.

The results obtained by determining binding capacity of IBL-102 antibody and IBL-101 antibody to each of the antigens as described above are shown in Table 2. The numerical values represent absorbance at 490 nm. IBL-102 antibody showed highly specific immunoreactivity to E22P-Aβ42 and low affinity to other antigens. This result indicates that IBL-102 antibody very specifically recognizes only Aβ42 having a turn structure at positions 22 and 23, whereas IBL-101 antibody weakly binds to E22V-Aβ42, wild-type Aβ42, wild-type Aβ40, and the like.

**[Table 2]**

| Antibody | *µ*g/mL | E22P, Aβ1-42 | E22V, Aβ1-42 | Aβ1-42 | Aβ1-40 | BSA |
|---|---|---|---|---|---|---|
| IBL-101 | 1 | 2.247 | 0.493 | 2.13 | 2.078 | 0.038 |
| | 0.5 | 1.921 | 0.256 | 1.756 | 1.808 | 0.039 |
| | 0.25 | 1.553 | 0.147 | 1.279 | 1.35 | 0.036 |
| | 0.125 | 1.151 | 0.08 | 0.924 | 0.97 | 0.037 |
| IBL-102 | 1 | 1.284 | 0.03 | 0.231 | 0.449 | 0.039 |
| | 0.5 | 0.833 | 0.016 | 0.154 | 0.292 | 0.039 |
| | 0.25 | 0.496 | 0.009 | 0.102 | 0.165 | 0.039 |
| | 0.125 | 0.301 | 0.006 | 0.069 | 0.106 | 0.039 |

### (Example 4) Diagnosis of patients with Alzheimer's disease using IBL-102 antibody

Among patients with dementia who visited the Department of Neurology at Kyoto Prefectural University of Medicine and whose spinal fluids were collected upon obtaining a written consent of the collection, patients who have diagnosed as AD based on the subsequent clinical course, diagnostic imaging, and the like were considered as AD patients. Patients whose spinal fluids were examined in the process of diagnosing various nervous system diseases and who have consented in writing to use of the samples for research purposes were considered as controls (healthy subject). Spinal fluid samples were taken according to the method as described below. Cerebral spinal fluids were collected directly into a sterile, individually packed tube (ASIAKIZAI Co., Ltd., PP screw tube 15 mL) by fasting lumbar puncture.

A 96-well plate was immobilized (coated) with anti-Aβ mouse monoclonal 82E1 antibody (an antibody recognizing N-terminus (amino acids at positions 1 to 16) of human Aβ; Horikoshi Y, et al., Biochem Biophys Res Commun. (2004) 319 (3): 733-7; Immuno-Biological Laboratories Co, Ltd., #10323; the same shall apply hereinafter) in 0.1 M carbonate buffer at 1 µg/well. One hundred µL of each of the sample, serially diluted standard (E22P Aβ-40 dimer), and a buffer for dilution (blank) was added to each well. After standing overnight at 4°C, the reaction solution was removed from wells, and the wells were washed. One hundred µL of anti-Aβ mouse monoclonal antibody IBL-102 labeled with HRP was added to each well. After standing for 60 minutes at 4°C, the reaction solution was removed from wells, and the wells were washed. One hundred µL of TMB substrate solution was added. After standing for 30 minutes at room temperature with protection from light, 100 µL of 1N H₂SO₄ was added to stop the color reaction. Absorbance at 450 nm was measured. A standard curve generated using the standard was used to determine the amount of Aβ having the toxic conformer in each sample.

The total amount of Aβ42 was determined by measuring the amount of Aβ42 in the same spinal fluid samples according to the method as previously reported. The amount of Aβ having the toxic conformer relative to the determined total amount of Aβ42 was calculated to analyze the correlation with development of AD.

The results obtained by measuring the substance bound to IBL-102 antibody (toxic conformer of Aβ: Toxic Aβ) in cerebral spinal fluids from patients with Alzheimer's disease (AD) and healthy subjects (Control) are shown in Figure 1. The results obtained by calculating the total amount of Aβ42 in the same cerebral spinal fluids, and the ratio of the amount of Aβ having the toxic conformer to the total amount of Aβ42 are shown in Figure 14. A high level of Toxic Aβ was detected with IBL-102 antibody in AD patients, whereas a low level of Toxic Aβ was detected in healthy subjects. The result of Mann Whitney test was P = 0.0635. Such a clear difference between AD patients and healthy subjects was not found when IBL-101 antibody was used (not shown). Moreover, the difference was demonstrated to be more remarkable when calculated as a ratio of the amount of Aβ having the toxic conformer to the total amount of Aβ42. In other words, it was shown that the ratio of the amount of Aβ having the toxic conformer to the total amount of Aβ42 in AD patients was significantly higher than that in non-AD patients (p < 0.05). Therefore, it was demonstrated that IBL-102 antibody was available for the diagnosis of AD.

### (Example 5) Effect of IBL-102 antibody on Aβ42-induced neuronal cell toxicity

To determine whether IBL-102 antibody can suppress cytotoxicity of Aβ42, Aβ42-induced neuronal cell toxicity was evaluated with an MTT assay in rat primary neurons (K. Murakami, et al. (2003) J. Biol. Chem., 278: 46179-46187). Cultured rat primary neurons were cytotoxically stimulated by adding 1 µM wild-type Aβ42 or E22P-Aβ42. The group without cytotoxic stimulation was defined as a control (no stimulation). After adding Aβ42 or E22P-Aβ42, each of 0.1 mg/mL IBL-102 antibody and IgG antibody (negative control) was added as a test agent. The group without antibody was defined as a control (no agent). The neurons were cultured for 4 days at 37°C. The concentration of Aβ used was set to 10⁻⁶ M, which is near the IC50 value of wild-type Aβ42. Statistically significant difference was determined by one-way analysis of variance followed by Tukey's test.

The results are shown in Figure 2. The rat primary neurons treated with 1 µM wild-type Aβ42 and E22P-Aβ42 showed a viability lower than in control cells. Addition of IBL-102 antibody inhibited cytotoxicity of Aβ42 and E22P-Aβ42. Accordingly, IBL-102 antibody was shown to markedly inhibit cytotoxicity of Aβ42 and the toxic conformer of Aβ.

### (Example 6) Immunostaining of brain from AD model mice (accelerated APP transgenic mice)

PS2, Tg2576 mouse (T. Toda, et al. (2011) J. Biomed. Biotech., 2011, 617974), which is generated by allowing APP transgenic mouse, Tg2576 (K. Hsiao, et al. (1996) Science, 274: 99-102) to overexpress presenilin 2, an enzyme producing Aβ42, was used as an AD model mouse. Brain was removed from the mice under diethyl ether anesthesia and fixed in 4% paraformaldehyde. The brain was embedded in paraffin and sliced into 5 µm thick sections. The sections were deparaffinized and hydrated followed by activation of antigens using formic acid and inactivation of endogenous peroxidase. The sections were then blocked with serum. The sections were then reacted with 1 µg/ml or 5 µg/ml 82E1 (positive control), 5 µg/ml or 20 µg/ml IBL-101, or 5 µg/ml or 20 µg/ml IBL-102 as a primary antibody overnight at 4°C, washed, and reacted with a biotinylated anti-mouse IgG antibody as a secondary antibody at room temperature. The intensity of immune reaction was enhanced using an avidin-biotin complex kit. The sections were stained with 3,3'-diaminobenzidine.

The results are shown in Figure 3. The positive control, 82E1 clearly stained senile plaques at 1 µg/ml and 5 µg/ml. IBL-101 stained brain parenchyma in addition to senile plaques at 5 µg/ml and stained more strongly at 20 µg/ml. On the other hand, IBL-102 stained no senile plaque and brain parenchyma at both 5 µg/ml and 20 µg/ml. It is demonstrated that unlike IBL-101, IBL-102 does not react with senile plaques and exhibits a characteristic reactivity.

### (Example 7) Efficacy evaluation test in AD model mice (accelerated APP transgenic mice)

PS2, Tg2576 was used as an AD model mouse. Antibody administration was started at 3 months, and a behavioral test was performed at 6 months provided that the day at which the mice were born was considered as day 0. IgG, IBL-101 antibody, and IBL-102 antibody were used for administration (n = 5 to 9 in each group). Each antibody was administered at a dose of 10 mg/kg once a week for 3 months.

Elevated plus-maze test and Nesting test were performed for behavioral evaluation of neurologic symptoms. Pathological evaluations were achieved by immunohistochemical staining for senile plaques (using 82E1 antibody) and measuring of the amount of Aβ in the brain (in soluble and insoluble fractions) in ELISA. The soluble and insoluble fractions were prepared according to a conventional method. Statistically significant difference was determined by one-way analysis of variance followed by Tukey's test.

The results of Elevated plus-maze test were shown in Figure 4. PS2, Tg2576 mice in the IgG-administered group showed a decreased preference for the closed arms. Such a neurologic symptom was not improved by administering IBL-101, but was markedly improved by administering IBL-102. These results revealed that high selectivity for the toxic conformer is important for the treatment of AD with the antibody, and highly selective IBL-102 has a marked therapeutic/prophylactic effect.

The results of Nesting test are shown in Figure 5. The Nesting test was performed by placing a mouse in a cage containing ten 6-cm square paper pieces for 4 days and then scoring nesting habits according to the following criteria. Score 0: no nesting; Score 1: gathering all strips at a corner; Score 2: gathering and processing all strips; Score 3: gathering and tearing to small pieces. PS2, Tg2576 mice in the IgG-administered group had lowered nesting scores. Such a neurologic symptom was not improved by administering IBL-101, but was improved by administering IBL-102. These results revealed that high selectivity for the toxic conformer is important for the treatment of AD with the antibody, and highly selective IBL-102 has a therapeutic/prophylactic effect.

The results of immunostaining of senile plaques are shown in Figure 6 (brain) and Figure 7 (hippocampus). The presence of senile plaques was detected in brain and hippocampus of PS2, Tg2576 mice in the IgG-administered group. The deposit of senile plaques was not inhibited by administering IBL-101 and IBL-102. These results revealed that IBL-101 and IBL-102 have no effect that reduces the number of senile plaques. IBL-102 appeared to improve neurologic symptoms independently of the number of senile plaques.

The results obtained by measuring the amount of Aβ in brain using ELISA are shown in Figure 8. Aβ40 and Aβ42 were detected in brain of PS2, Tg2576 mice in the IgG-administered group. This Aβ40 and Aβ42 were not decreased by administering IBL-101 and IBL-102. These results revealed that IBL-101 and IBL-102 have no effect that reduces the total amount of Aβ40 and Aβ42 in brain. IBL-102 appeared to improve neurologic symptoms independently of the total amount of Aβ40 and Aβ42.

### (Example 8) Sequencing of IBL-102 antibody

Cloning of heavy chain (hereinafter referred to as "Hc") and light chain (hereinafter referred to as "Lc") was performed to determine the gene sequence of IBL-102 antibody. mRNA was extracted from a hybridoma producing IBL-102 antibody, and cDNA was prepared. 5'-Rapid amplification cDNA end (5'-RACE) was performed according to a conventional method to determine the base sequence of 5' end portion of Hc and Lc genes. Primers specific for Hc and Lc were used to clone the full-length cDNAs of Hc and Lc genes, respectively. The determined sequences of Hc and Lc genes are as described in Figure 9 (SEQ ID NO: 1 and SEQ ID NO: 8), and the amino acid sequences of Hc and Lc (including signal sequence) are as described in Figure 10 (SEQ ID NO: 2 and SEQ ID NO: 9). The VH and VL sequences of IBL-102 antibody are as set forth in SEQ ID NO: 4 and SEQ ID NO: 11, respectively. The sequences of CDRH1, CDRH2, CDRH3, CDRL1, CDRL2, and CDRL3 of IBL-102 antibody are as set forth in SEQ ID NO: 5, 6, 7, 12, 13, and 14, respectively.

### (Example 9) Determination of dissociation constant using BIACORE

The dissociation constant (KD) between IBL-102 or IBL-101 antibody and the antigens recognized by these antibodies was measured using BIACORE (GE Healthcare Bioscience, BIACORE-X100). According to the BIACORE manual (GE Healthcare Bioscience, BIACORE-X100), biotinylated E22P-Aβ42 or biotinylated wild-type Aβ42 was immobilized on an SA CHIP, and IBL-101 antibody or IBL-102 antibody was flowed over the chip. The association rate constant Ka1 and the dissociation rate constant Kd1 were measured, and the value of association constant KD was determined using the bivalent fitting.

The results of Ka1, Kd1, and KD values are shown in Table 3 below. As shown below, the binding of IBL-102 antibody to E22P-Aβ42 and E22P-Aβ42 dimer was stronger than the binding of IBL-101 antibody to E22P-Aβ42 and E22P-Aβ42 dimer.
[Table 3]

**Table 3. The dissociation constant (K_{D}) together with the association (kₐ) and dissociation (k_{d}) rate constants of IBL-102 and IBL-101 for Aβ derivatives.**

| Antibody | Immobilized Aβ | *k*ₐ (M⁻¹ s⁻¹) | *k*_{d} (s⁻¹) | *K*_{D} (nM) |
|---|---|---|---|---|
| **IBL-202** | Aβ42 | <5.0 x 10⁴ | >5.0 x 10⁻⁷ | >100 |
| | E22P-Aβ42 | 2.3 x 10⁵ | 7.1 x 10⁻⁴ | 3.1 |
| | E22P-Aβ42 dimer | 2.3 x 10⁵ | 3.9 x 10⁻⁴ | 1.7 |
| **IBL-101** | Aβ42 | 3.4 x 10⁴ | 4.9 x 10⁻⁴ | 14 |
| | E22P-Aβ42 | 4.0 x 10⁴ | 9.2 x 10⁻⁴ | 23 |
| | E22P-Aβ42 dimer | 9.5 x 10² | 1.1 x 10⁻⁴ | 11 |

### (Example 10) Determination of binding capacity to Aβ polymer

Aβ is believed to have toxicity due to oligomerization by binding together. To investigate the binding of IBL-102 antibody to Aβ oligomers, binding capacity to each of the following antigens was determined by an enzyme immunoassay: wild-type Aβ42; E22P-Aβ42; E22P, V40L, LDap-Aβ42 dimer; and E22P,A"30L, LDap-Aβ40 dimer. Each antigen in a buffer for immobilization (0.1 M carbonate buffer, pH 9.5) was added to a 96-well ELISA plate at 50 ng/well and allowed to react overnight at 4°C to achieve immobilization. After washing with PBS, the plate was blocked with a blocking solution (1%BSA, 0.05%Tween 20/PBS). After each antigen was immobilized as described above, IBL-102 antibody was diluted to 1, 0.5, 0.25, and 0.125 µg/mL and added to each of the immobilized antigens at 100 µL/well. After reacting for 60 minutes at room temperature, the plate was washed, and a labeled antibody (Anti-Mouse IgG (H + L) Goat Fab'-HRP) was added at 50 µL/well. The reaction was continued for 60 minutes at room temperature, and the plate was then washed. A substrate solution for color reaction (o-phenylenediamine dihydrochloride; OPD) was added to trigger the color reaction (for 30 minutes at room temperature with protection from light), and a stop solution (2N H2SO4) was added to stop the reaction. Subsequently, absorbance at 492 nm was measured. IBL-101 antibody and 4G8 antibody (a monoclonal antibody having an epitope at 18 to 22 residues of Aβ sequence (not the turn structure portion); H. M. Wisniewski, et al. (1989) Acta. Neuropathol., 78: 22-27) were similarly tested as a control.

The results are shown in Figure 11. IBL-101 antibody equally bound to all of wild-type Aβ42, E22P-Aβ42 monomer, E22P, V40L, LDap-Aβ42 dimer, and E22P, V40L, LDap-Aβ40 dimer with low avidity. On the other hand, IBL-102 antibody bound to E22P-Aβ42 monomer strongly and further bound to E22P, V40L, LDap-Aβ42 dimer and E22P, V40L, LDap-Aβ40 dimer more strongly although IBL-102 antibody has the avidity to wild-type Aβ42 as strong as the avidity of IBL-101 to wild-type Aβ42. 4G8 antibody, which was used as a control, bound to wild-type Aβ42 strongly, but bound to E22P-mutated Aβ42 weakly. This indicates that IBL-102 antibody specifically and strongly binds to Aβ oligomers.

### (Example 11) Single dose study of vaccine

To investigate effects of a single intravenous dose of IBL-102 antibody, IBL-102 antibody was administered to AD model mice Tg2576 aged 18 months. The schedule of experiment is shown in Figure 12. IBL-102 antibody (10 to 20 mg/kg) or PBS was alternately administered twice in total at an interval of 1 week from the first administration of antibody. The mice were divided into two groups, group A and group B (n = 7 in each group). Group A received IBL-102 antibody at the first dose and PBS at the second dose, while group B received PBS at the first dose and IBL-102 antibody at the second dose. A behavioral test was performed before (pre-test) and after (post-test) the first and second doses to investigate effects of antibody administration. In the behavioral test, the duration of contact with a novel object (duration of sniff) was measured, and curiosity index was scored by comparing with control mice (young B6). Specifically, re-exploring score was determined by dividing (the number of contact in the post-test) / (the number of contact in the pre-test) of each individual in the IBL-102-administered group or PBS-administered group by the average of (the number of contact in the post-test) / (the number of contact in the pre-test) in the control mice group. Different objects were used in the behavioral tests for the first and second doses.

The results are shown in Figure 13. The number of mice in the PBS-administered group that forgot contacting with the object once in the pre-test and recognized the same object to be a novel object and exhibited seeking behavior again in the post-test was greater than the number in control group, while administration of IBL-102 antibody increased the number of mice that did not contact the object that the mice had contacted once in the pre-test. This indicates that administration of IBL-102 antibody improves memory impairment due to Aβ.

### (Example 12) Determination #3 of binding specificity of IBL-102 antibody

The binding capacity of IBL-102 antibody and IBL-101 antibody to each of the following antigens was determined and compared by an enzyme immunoassay: wild-type Aβ40, wild-type Aβ42, E22P-Aβ42, E22V-Aβ42, G33P-Aβ42, L34P-Aβ42, L34P-Aβ42, V36P-Aβ42, G37P-Aβ42, G38P-Aβ42, V39P-Aβ42, V40P-Aβ42, and I41P-Aβ42. Specifically, each of the antigens as described above was added at 2.5 µg/well to a 96-well plate immobilized (coated) with 82E1 antibody (recognizing residues at positions 1 to 16 of Aβ sequence (not the turn structure portion); see Y. Horikoshi, et al. (2004) Biochem Biophys Res Commun. 2004 Jul 2; 319 (3): 733-7.; the same shall apply hereinafter) in 0.1 M carbonate buffer at 1 µg/well. After standing for 2 hours at room temperature, the reaction solution was removed from wells. After washing, the wells were blocked overnight at 4°C. One hundred µL of anti-Aβ mouse monoclonal antibody IBL-101 and HRP-labeled anti-Aβ mouse monoclonal antibody IBL-102 were added to each well (at 120, 60, 30, and 15 ng/mL). After standing for 60 minutes at room temperature, the reaction solution was removed from wells. After washing, an HRP-labeled secondary antibody was added for one hour at room temperature. One hundred µL of o-phenylenediamine (OPD) substrate solution was added. After standing for 30 minutes at room temperature with protection from light, 50 µL of 2M H₂SO₄ was added to stop the color reaction. Absorbance at 492 nm was measured.

The results obtained by determining and comparing the binding capacities of IBL-102 antibody and IBL-101 antibody to each of the antigens as described above are shown in Figure 15. The numerical values represent absorbance at 492 nm. IBL-102 antibody showed highly specific immunoreactivity to E22P-Aβ42 and low affinity to other antigens. This result indicates that IBL-102 antibody very specifically recognizes only Aβ42 having a turn structure at positions 22 and 23.

### (Example 13) Single dose study of vaccine (IgG control)

To investigate effects of a single intravenous dose of IBL-102 antibody, IBL-102 antibody was administered to AD model mice Tg2576 aged 16 to 19 months. Wild-type mice and Tg2576 mice were allowed to memorize two objects A1 and A2, which are very similar, for 10 minutes per day for 3 days. Next day, wild-type mice received PBS, and Tg2576 mice received IgG or IBL-102 (20 mg/kg). On the day following the administration, the numbers of which the mice contacted with one (A1) of the two memorized objects A1 and A2, and a novel object B were counted. The mice were evaluated for memory using (the number of contacting with object A1) / ((the number of contacting with object A1) + (the number of contacting with object B)) and (the number of contacting with object B) / ((the number of contacting with object A1) + (the number of contacting with object B)) as an indicator.

The results are shown in Figure 16. Wild-type mice showed a significantly high preference for the novel object (Novel: B) (p = 0.0003), while IgG-administered Tg2576 mice did not show the preference and showed decreased memory. On the other hand, IBL-102-administered Tg2576 mice showed a significant preference for the novel object (p = 0.0002) and showed no decreased memory. This indicates that administration of IBL-102 antibody improves memory impairment due to Aβ.

## Claims

1. An antibody or immunoreactive fragment thereof that highly specifically recognizes Aβ having a turn structure at amino acid positions 22 and 23 but does not recognize Aβs having other structures.

2. The antibody or immunoreactive fragment thereof of claim 1, further recognizing Aβ oligomers.

3. The antibody or immunoreactive fragment thereof of claim 2, wherein the Aβ oligomers are dimer.

4. The antibody or immunoreactive fragment thereof of any one of claims 1 to 3, wherein the Aβ is Aβ42.

5. The antibody or immunoreactive fragment thereof of any one of claims 1 to 4, wherein the other structures of Aβ includes at least one or more structures selected from the group consisting of a turn structure at amino acid positions 21 and 22 of Aβ, a turn structure at amino acid positions 23 and 24 of Aβ, a turn structure at amino acid positions 24 and 25 of Aβ, a turn structure at amino acid positions 25 and 26 of Aβ, and a turn structure at amino acid positions 35 and 36 of Aβ.

6. An antibody or immunoreactive fragment thereof that highly specifically recognizes an epitope which is bound by an antibody in which VH has the amino acid sequence of SEQ ID NO: 4 and VL has the amino acid sequence of SEQ ID NO: 11, but does not recognize an epitope which is not bound by an antibody in which VH has the amino acid sequence of SEQ ID NO: 4 and VL has the amino acid sequence of SEQ ID NO: 11.

7. An antibody or immunoreactive fragment thereof that competitively inhibits binding of an antibody in which VH has the amino acid sequence of SEQ ID NO: 4 and VL has the amino acid sequence of SEQ ID NO: 11 to its antigen, and does not bind to an epitope which is not bound by an antibody in which VH has the amino acid sequence of SEQ ID NO: 4 and VL has the amino acid sequence of SEQ ID NO: 11.

8. An antibody or immunoreactive fragment thereof wherein CDR1, CDR2, and CDR3 of heavy chain variable region have the amino acid sequences of SEQ ID NO: 5, SEQ ID NO: 6, and SEQ ID NO: 7, respectively.

9. The antibody or immunoreactive fragment thereof of claim 8, wherein furthermore, CDR1, CDR2, and CDR3 of light chain variable region have the amino acid sequences of SEQ ID NO: 12, SEQ ID NO: 13, and SEQ ID NO: 14, respectively.

10. The antibody or immunoreactive fragment thereof of any one of claims 1 to 7, wherein the CDR1, CDR2, and CDR3 of heavy chain variable region have the amino acid sequences of SEQ ID NO: 5, SEQ ID NO: 6, and SEQ ID NO: 7, respectively.

11. The antibody or immunoreactive fragment thereof of claim 10, wherein furthermore, the CDR1, CDR2, and CDR3 of light chain variable region have the amino acid sequences of SEQ ID NO: 12, SEQ ID NO: 13, and SEQ ID NO: 14, respectively.

12. An antibody or immunoreactive fragment thereof, wherein VH has the amino acid sequence of SEQ ID NO: 4.

13. The antibody or immunoreactive fragment thereof of claim 12, wherein furthermore, VL has the amino acid sequence of SEQ ID NO: 11.

14. A nucleic acid molecule encoding an antibody or immunoreactive fragment thereof having the amino acid sequence of SEQ ID NO: 4.

15. The nucleic acid molecule of claim 14, consisting of the nucleotide sequence at from position 126 to position 479 of SEQ ID NO: 1.

16. A nucleic acid molecule encoding an antibody or immunoreactive fragment thereof having the amino acid sequence of SEQ ID NO: 11.

17. The nucleic acid molecule of claim 16, consisting of the nucleotide sequence at from position 130 to position 465 of SEQ ID NO: 8.

18. A vector capable of expressing an antibody or immunoreactive fragment thereof having the amino acid sequence of SEQ ID NO: 4, and/or an antibody or immunoreactive fragment thereof having the amino acid sequence of SEQ ID NO: 11.

19. The vector of claim 18, comprising the nucleic acid molecule of claim 14 or 15, and/or the nucleic acid molecule of claim 16 or 17.

20. A host cell comprising the vector of claim 18 or 19.

21. A pharmaceutical composition comprising the antibody or immunoreactive fragment thereof of any one of claims 1 to 13.

22. The pharmaceutical composition of claim 21, for preventing, treating, or improving AD.

23. The pharmaceutical composition of claim 21, for improving cognitive function of AD patients.

24. The pharmaceutical composition of claim 21, for improving memory impairment of AD patients.

25. A diagnostic composition comprising the antibody or immunoreactive fragment thereof of any one of claims 1 to 13.

26. The diagnostic composition of claim 25, for diagnosis of AD.

27. A kit for measuring a toxic conformer of Aβ, comprising the antibody or immunoreactive fragment thereof of any one of claims 1 to 13.

28. A composition for measurement of the toxic conformer of Aβ, comprising the antibody or immunoreactive fragment thereof of any one of claims 1 to 13.

29. A method for measuring a level of the toxic conformer of Aβ in a sample, comprising the step of contacting the sample with the antibody or immunoreactive fragment thereof of any one of claims 1 to 13.

30. A method for diagnosing AD, comprising the steps of:
a) contacting a sample from a subject with the at least one antibody or immunoreactive fragment thereof of any one of claims 1 to 13 in vitro,
b) measuring a level of an antigen bound to the antibody or immunoreactive fragment thereof, and
c) diagnosing the presence or absence of AD, or stage of AD in the subject from the measured level of the antigen,
wherein the subject is diagnosed to likely have AD when the measured level of an antigen bound to the antibody or immunoreactive fragment thereof of any one of claims 1 to 13 is higher than the level in a sample from a healthy subject.

31. A method for evaluating AD, comprising the steps of:
a) contacting a sample from a subject with the at least one antibody or immunoreactive fragment thereof of any one of claims 1 to 13,
b) measuring a level of an antigen bound to the antibody or immunoreactive fragment thereof,
c) contacting the sample from the subject with an anti-Aβ antibody which does not specifically recognize the structure of Aβ, and measuring the amount of the bound antibody to determine total amount of Aβ in the sample from the subject, and
d) calculating the ratio of the level of the antigen bound to the antibody or immunoreactive fragment thereof of any one of claims 1 to 13 to the level of total Aβ in the sample from the subject, and evaluating the presence or absence of AD, or stage of AD in the subject from the determined ratio,
wherein the subject is evaluated likely to have AD when the ratio of the level of the antigen bound to the antibody or immunoreactive fragment thereof of any one of claims 1 to 13 to the level of total Aβ is higher than the ratio in a sample from a healthy subject.

32. A method for determining the presence or absence of the toxic conformer of amyloid β in a sample, comprising the steps of:
(a) contacting the sample with the antibody or immunoreactive fragment thereof of any one of claims 1 to 13,
(b) detecting the toxic conformer of amyloid β bound to the antibody or immunoreactive fragment thereof, and
(c) determining the presence of the toxic conformer of amyloid β in the sample when the toxic conformer of amyloid β is detected in the previous step, or determining the absence of the toxic conformer of amyloid β in the sample when the toxic conformer of amyloid β is not detected in the previous step.

33. A method for determining a level of the toxic conformer of amyloid β in a sample, comprising the steps of:
(a) contacting the sample with the antibody or immunoreactive fragment thereof of any one of claims 1 to 13,
(b) measuring the amount of the toxic conformer of amyloid β bound to the antibody or immunoreactive fragment thereof, and
(c) calculating the level of the toxic conformer of amyloid β in the sample from the measured amount of the toxic conformer.
